# EUROPEAN PATENT SPECIFICATION

(11) **EP 3 096 779 B1**
(45) Date of publication and mention of the grant of the patent: **18.12.2019**
(21) Application number: 15708578.8
(22) Date of filing: 15.01.2015
(51) Int. Cl.: A61K 38/48, A61P 7/04

(54) **COMPOSITIONS AND METHODS FOR TREATING INTRACEREBRAL HEMORRHAGE**
ZUSAMMENSETZUNGEN UND VERFAHREN ZUR BEHANDLUNG VON INTRAZEREBRALER BLUTUNG
COMPOSITIONS ET MÉTHODES POUR LE TRAITEMENT DE L'HÉMORRAGIE INTRACÉRÉBRALE

(30) Priority: 24.01.2014 US 201461931071 P
(43) Date of publication of application: 30.11.2016
(73) Proprietor: Pfizer Inc., New York, NY 10017 (US)
(72) Inventor: ARKIN, Steven, Belmont, Massachusetts 02478 (US); FRUEBIS, Joachim, Bedford, Massachusetts 01730 (US); CARR, Marcus E., Holland, Pennsylvania 18966 (US); HETT, Sunita, Cambridge, Massachusetts 02138 (US); JASUJA, Reema, Cambridge, Massachusetts 02140 (US); PITTMAN, Debra D., Windham, New Hampshire 03087 (US)
(74) Representative: Pfizer
(86) International application number: PCT/IB2015/050313
(87) International publication number: WO 2015/110939

(56) References cited:
- WO-A1-2011/008885
- WO-A1-2013/049804
- WO-A1-2014/018120
- WO-A1-2014/118677
- WO-A2-2007/059513
- WO-A2-2009/090240
- MAYER S A ET AL: "Ultra-early hemostatic therapy for acute intracerebral hemorrhage", SEMINARS IN HEMATOLOGY, PHILADELPHIA, PA, US, vol. 43, no. Suppl. 1, 1 January 2006 (2006-01-01), pages S70-S76, XP009120241, ISSN: 0037-1963, DOI: 10.1053/J.SEMINHEMATOL.2005.11.020
- Stephan A Mayer ET AL: "n Recombinant Activated Factor VII for Acute Intracerebral Hemorrhage for the Recombinant Activated Factor VII Intracerebral Hemorrhage Trial Investigators* From the background", N Engl J Med, 1 January 2005 (2005-01-01), pages 777-85, XP55184485, Retrieved from the Internet: URL:http://www.nejm.org/doi/pdf/10.1056/NE JMoa042991 [retrieved on 2015-04-21]
- MAYER STEPHAN A ET AL: "Efficacy and safety of recombinant activated factor VII for acute intracerebral hemorrhage", NEW ENGLAND JOURNAL OF MEDICINE, MASSACHUSETTS MEDICAL SOCIETY, BOSTON, MA, US, vol. 358, no. 20, 15 May 2008 (2008-05-15) , pages 2127-2137, XP002537672, ISSN: 1533-4406, DOI: 10.1056/NEJMOA0707534
- SUDHIR KUMAR: "Recombinant activated factor VII for acute intracerebral hemorrhage", INDIAN JOURNAL OF CRITICAL CARE MEDICINE, vol. 9, no. 1, 1 January 2005 (2005-01-01) , page 11, XP55184291, ISSN: 0972-5229, DOI: 10.4103/0972-5229.16262
- LU G ET AL: "Reconstructed recombinant factor Xa as an 5 antidote to reverse anticoagulation by factor Xa inhibitors", JOURNAL OF THROMBOSIS AND HAEMOSTASIS, BLACKWELL PUBLISHING, OXFORD, GB, vol. 7, no. suppl. 2, 1 July 2009 (2009-07-01), pages 309/OC-TH, XP009177312, ISSN: 1538-7933
- HOLLENBACH S ET AL: "Bolus administration of PRT064445, a recombinant Factor Xa inhibitor antidote, reverses blood loss and PD markers in a rat model following enoxaparin induced anticoagulation", EUROPEAN HEART JOURNAL, OXFORD UNIVERSITY PRESS, GB, vol. 33, no. Suppl, 1 January 2012 (2012-01-01), pages 309-310, XP009177324, ISSN: 0195-668X
- LACRAMIOARA IVANCIU ET AL: "A zymogen-like factor Xa variant corrects the coagulation defect in hemophilia", NATURE BIOTECHNOLOGY, vol. 29, no. 11, 23 October 2011 (2011-10-23), pages 1028-1033, XP055111224, US ISSN: 1087-0156, DOI: 10.1038/nbt.1995
- NABIL K THALJI ET AL: "Reversal of Direct Factor Xa Inhibitors Using Factor Xa Zymogen-Like Variants", 28TH ANNUAL MD/PHD STUDENT CONFERENCE, KEYSTONE, COLORADO, JULY 26-28, 2013,, 27 July 2013 (2013-07-27), page 16, XP009177338,
- NABIL K THALJI ET AL: "Zymogen-Like FXa Is An Effective Pro-Hemostatic To Reverse The Anticoagulant Effects Of Direct FXa Inhibitors", INTERNET CITATION, [Online] page 1, XP002722746, Retrieved from the Internet: URL:https://ash.confex.com/ash/2013/webpro gram/Paper63599.html> [retrieved on 2014-04-01]
- TOSO RAFFAELLA ET AL: "The conformational switch from the factor X zymogen to protease state mediates exosite expression and prothrombinase assembly", JOURNAL OF BIOLOGICAL CHEMISTRY, AMERICAN SOCIETY FOR BIOCHEMISTRY AND MOLECULAR BIOLOGY, US, vol. 283, no. 27, 1 July 2008 (2008-07-01) , pages 18627-18635, XP002526299, ISSN: 0021-9258, DOI: 10.1074/JBC.M802205200
- ARNE LAUER ET AL: "Intracerebral haemorrhage associated with antithrombotic treatment: translational insights from experimental studies", THE LANCET NEUROLOGY, vol. 12, no. 4, 1 April 2013 (2013-04-01), pages 394-405, XP055184564, ISSN: 1474-4422, DOI: 10.1016/S1474-4422(13)70049-8

### SEQUENCE LISTING

<110> PFIZER INC.
<120> COMPOSITIONS AND METHODS FOR TREATING INTRACEREBRAL HEMORRHAGE
<130> PC072056A
<150> US 61/931,071
   <151> 2014-01-24
<160> 2
<170> PatentIn version 3.5
<210> 1
   <211> 488
   <212> PRT
   <213> Homo sapiens
<400> 1
<210> 2
   <211> 1560
   <212> DNA
   <213> Homo sapiens
<400> 2
   gactttgctc cagcagcctg tcccagtgag gacagggaca cagtactcgg ccacaccatg      60
   gggcgcccac tgcacctcgt cctgctcagt gcctccctgg ctggcctcct gctgctcggg     120
   gaaagtctgt tcatccgcag ggagcaggcc aacaacatcc tggcgagggt cacgagggcc     180
   aattcctttc ttgaagagat gaagaaagga cacctcgaaa gagagtgcat ggaagagacc     240
   tgctcatacg aagaggcccg cgaggtcttt gaggacagcg acaagacgaa tgaattctgg     300
   aataaataca aagatggcga ccagtgtgag accagtcctt gccagaacca gggcaaatgt     360
   aaagacggcc tcggggaata cacctgcacc tgtttagaag gattcgaagg caaaaactgt     420
   gaattattca cacggaagct ctgcagcctg gacaacgggg actgtgacca gttctgccac     480
   gaggaacaga actctgtggt gtgctcctgc gcccgcgggt acaccctggc tgacaacggc     540
   aaggcctgca ttcccacagg gccctacccc tgtgggaaac agaccctgga acgcaggaag     600
   aggtcagtgg cccaggccac cagcagcagc ggggaggccc ctgacagcat cacatggaag     660
   ccatatgatg cagccgacct ggaccccacc gagaacccct tcgacctgct tgacttcaac     720
   cagacgcagc ctgagagggg cgacaacaac ctcaccagga tcgtgggagg ccaggaatgc     780
   aaggacgggg agtgtccctg gcaggccctg ctcatcaatg aggaaaacga gggtttctgt     840
   ggtggaacca ttctgagcga gttctacatc ctaacggcag cccactgtct ctaccaagcc     900
   aagagattca aggtgagggt aggggaccgg aacacggagc aggaggaggg cggtgaggcg     960
   gtgcacgagg tggaggtggt catcaagcac aaccggttca caaaggagac ctatgacttc    1020
   gacatcgccg tgctccggct caagaccccc atcaccttcc gcatgaacgt ggcgcctgcc    1080
   tgcctccccg agcgtgactg ggccgagtcc acgctgatga cgcagaagac ggggattgtg    1140
   agcggcttcg ggcgcaccca cgagaagggc cggcagtcca ccaggctcaa gatgctggag    1200
   gtgccctacg tggaccgcaa cagctgcaag ctgtccagca gcttcatcat cacccagaac    1260
   atgttctgtg ccggctacga caccaagcag gaggatgcct gccaggggga cagcgggggc    1320
   ccgcacgtca cccgcttcaa ggacacctac ttcgtgacag gcatcgtcag ctggggagag    1380
   ggctgtgccc gtaaggggaa gtacgggatc tacaccaagg tcaccgcctt cctcaagtgg    1440
   atcgacaggt ccatgaaaac caggggcttg cccaaggcca agagccatgc cccggaggtc    1500
   ataacgtcct ctccattaaa gtgagatccc actcaaaaaa aaaaaaaaaa aaaaaaaaaa    1560

## Description

### CROSS-REFERENCE TO RELATED APPLICATION

This application claims the benefit of U.S. Provisional Application No. 61/931,071, filed 24 Jan 2014.

### REFERENCE TO SEQUENCE LISTING

The Sequence Listing submitted concurrently herewith under 37 CFR §1.821 in a computer readable form (CRF) via EFS-Web as file name PC072056A_SEQLIST_ST25.txt. The electronic copy of the Sequence Listing was created on 06 Jan 2015, with a file size of 7 kilobytes.

### BACKGROUND OF THE INVENTION

Effective therapies are needed to control excessive bleeding in a range of clinical conditions where bleeding cannot be controlled by surgical intervention or other means. One of the most devastating clinical syndromes involving uncontrolled bleeding is intracerebral hemorrhage (ICH). Non-traumatic ICH is bleeding into the parenchyma of the brain that may extend into the ventricles. At least ten percent of stroke cases worldwide are due to ICH. Estimates for the number of individuals affected annually in the United States range from 37,000 - 52,400 to as many as 79,500 (Qureshi, et al, N Engl J Med 344(19):1450-1460 (2001); Roger, et al, Circulation 125:e2-220 (2012)) and the incidence is expected to increase as the population ages. ICH continues to be associated with a poor clinical outcomes. Approximately 40-50% of patients die within one month (Qureshi; Broderick, et al, Stroke 38:2001-2023 (2007)) and a large proportion of affected individuals fail to regain functional independence by six months (Broderick). ICH is therefore a major cause of permanent partial or complete disability.

Hematoma volume following intracerebral hemorrhage has been shown to be an independent predictor of mortality and morbidity in patients with ICH (Broderick, et al, Stroke 24:987-993 (1993)). Hematoma volume expansion occurs in approximately 26% of intracerebral hemorrhage patients within 1 hour of the baseline CT scan (Brott, et al, Stroke 28:1-5 (1997)) and hematoma expansion is an additional independent predictor of mortality and poor patient outcome (Davis, etal, Neurology 66:1175-1181 (2006); Delcourt, etal, Neurology 79:314-319 (2012)).

There are no approved therapies for treatment of ICH and current management is largely supportive (Rincon and Mayer, Critical Care 12:237-250 (2008)). For example, administration of recombinant clotting factor VIIa reduced hematoma expansion in ICH patients, but did not improve neurological function at 90 days (Mayer, et al, NEJM 358:2127-2137 (2008)). Accordingly, to reduce mortality and improve functional neurologic outcomes, there exists a compelling need in the art for effective therapies to prevent ICH in susceptible patients and to treat patients with ICH.

### SUMMARY OF THE INVENTION

Applicants have addressed this critical unmet clinical need by providing compositions for use in preventing and/or treating ICH, including compositions for use in preventing and/or treating ICH. Particular non-limiting embodiments of the invention are set forth in the following paragraphs. The present invention is as described and set out in the claims, in particular the present invention provides; a Factor Xa (FXa) variant for use in the treatment of intracerebral haemorrhage (ICH), wherein Factor Xa (FXa) variant includes at least one substitution mutation selected from the group consisting of (a) the amino acid at the position corresponding to 235 in SEQ ID NO:1 is substituted with Thr, Leu, Phe, Asp or Gly; and (b) the amino acid at the position corresponding to 236 in SEQ ID NO:1 is substituted with Leu, Ala, or Gly.

According to certain embodiments, the disclosure provides a Factor Xa (FXa) variant for use in treating intracerebral hemorrhage (ICH) in a subject by administering to the subject a therapeutically effective amount of a Factor Xa (FXa) variant including at least one substitution mutation at the amino acid position corresponding to 235 or 236 in SEQ ID NO:1. Also disclosed are FXa variants including at least one substitution mutation at the amino acid position corresponding to 235 and/or 236 in SEQ ID NO:1 for use in treating intracerebral hemorrhage (ICH) in a subject. In some disclosed embodiments, the substitution at the position corresponding to position 235 can be with Thr, Leu, Phe, Asp or Gly. In other disclosed embodiments, the substitution at the position corresponding to position 236 can be with Leu, Ala, or Gly.

According to certain embodiments, the disclosure provides a Factor Xa (FXa) variant for use in reducing the likelihood that a subject with ICH will die as a result of ICH by administering to the subject a therapeutically effective amount of a Factor Xa (FXa) variant including at least one substitution mutation at the amino acid position corresponding to 235 or 236 in SEQ ID NO:1. In some disclosed embodiments, the substitution at the position corresponding to position 235 can be with Thr, Leu, Phe, Asp or Gly. In other disclosed embodiments, the substitution at the position corresponding to position 236 can be with Leu, Ala, or Gly.

According to certain embodiments, the disclosure provides a Factor Xa (FXa) variant for use in improving the brain function of a subject after the subject has suffered ICH by administering to the subject a therapeutically effective amount of a Factor Xa (FXa) variant including at least one substitution mutation at the amino acid position corresponding to 235 or 236 in SEQ ID NO:1. In some disclosed embodiments, the substitution at the position corresponding to position 235 can be with Thr, Leu, Phe, Asp or Gly. In other disclosed embodiments, the substitution at the position corresponding to position 236 can be with Leu, Ala, or Gly. According to some disclosed embodiments, improved brain function is associated with reducing elevated intracranial pressure (ICP) in the subject caused by ICH to or below a pressure of 80 mm Hg, 70 mm Hg, 60 mm Hg, 50 mm Hg, 40 mm Hg, 30 mm Hg, 20 mm Hg, or 10 mm Hg. In other disclosed embodiments, improved brain function is associated with maintaining cerebral perfusion pressure (CPP) at or above a pressure of 40 mm Hg, 50 mm Hg, 60 mm Hg, 70 mm Hg, 80 mm Hg, 90 mm Hg, 100 mm Hg, 110, mm Hg, or 120 mm Hg. In other disclosed embodiments, improved brain function is associated with maintaining brain tissue oxygen tension (PbtO2) at or above a pressure of 6 mm Hg, 8 mm Hg, 10 mm Hg, 12 mm Hg, 14 mm Hg, 16 mm Hg, 18 mm Hg, 20, mm Hg, 22 mm Hg, or 24 mm Hg. In other disclosed embodiments, improved brain function is associated with reducing the ratio of the concentrations of lactate to pyruvate (LAR) to or below 60, 50, 40, 30, or 20. In other disclosed embodiments, improved brain function is associated with reducing the ratio of the cerebrovascular pressure reactivity index (PRx) to or below 0.5, 0.4, 0.3, 0.2, 0.1, 0.0, -0.1, -0.2, or -0.3.

According to certain embodiments, the disclosure provides a Factor Xa (FXa) variant for use in reducing neurological impairment of a subject after the subject has suffered ICH by administering to the subject a therapeutically effective amount of a Factor Xa (FXa) variant including at least one substitution mutation at the amino acid position corresponding to 235 or 236 in SEQ ID NO:1. In some disclosed embodiments, the substitution at the position corresponding to position 235 can be with Thr, Leu, Phe, Asp or Gly. In other disclosed embodiments, the substitution at the position corresponding to position 236 can be with Leu, Ala, or Gly. According to some disclosed embodiments, reduced neurological impairment is assessed using the Glasgow Coma Score and as a result of treatment the subject's score improves from 3 to 4 or higher, from 4 to 5 or higher, from 5 to 6 or higher, from 6 to 7 or higher, from 7 to 8 or higher, from 8 to 9 or higher, from 9 to 10 or higher, from 10 to 11 or higher, from 11 to 12 or higher, from 12 to 13 or higher, from 13 to 14 or higher, or from 14 to 15. In other disclosed embodiments, neurological impairment is assessed using the Glasgow Outcome Scale extended version and as a result of treatment the average score of treated subjects improves to 1 or better, 2 or better, 3 or better, 4 or better, 5 or better, 6 or better, 7 or better, or 8. In other disclosed embodiments, neurological impairment is assessed using the Barthel Index and as a result of treatment the average score of treated subjects improves to 10 or better, 20 or better, 30 or better, 40 or better, 50 or better, 60 or better, 70 or better, or 80 or better, or 90 or better. In other disclosed embodiments, neurological impairment is assessed using the NIH Stroke Scale and as a result of treatment the average score of treated subjects is reduced from 42 to 41 or less, 41 to 40 or less, 40 to 39 or less, 39 to 38 or less, 38 to 37 or less, 37 to 36 or less, 36 to 35 or less, 35 to 34 or less, 34 to 33 or less, 33 to 32 or less, 32 to 31 or less, 31 to 30 or less, 30 to 29 or less, 29 to 28 or less, 28 to 27 or less, 27 to 26 or less, 26 to 25 or less, 25 to 24 or less, 24 to 23 or less, 23 to 22 or less, 22 to 21 or less, 21 to 20 or less, 20 to 19 or less, 19 to 18 or less, 18 to 17 or less, 17 to 16 or less, 16 to 15 or less, 15 to 14 or less, 14 to 13 or less, 13 to 12 or less, 12 to 11 or less, 11 to 10 or less, 10 to 9 or less, 9 to 8 or less, 8 to 7 or less, 7 to 6 or less, 6 to 5 or less, 5 to 4 or less, 4 to 3 or less, 3 to 2 or less, 2 to 1 or less, or 1 to 0. In other disclosed embodiments, neurological impairment is assessed using the modified Rankin Scale and as a result of treatment the average score of treated subjects is reduced from between about 5 and 6 to between about 4 and 5, from between about 4 and 5 to between about 3 and 4, from between about 3 and 4 to between about 2 and 3, from between about 2 and 3 to between about 1 and 2, or from between about 1 and 2 to between about 0 and 1. In other disclosed embodiments, neurological impairment is assessed using the modified Rankin Scale and as a result of treatment the proportion of treated subjects with a modified Rankin Scale score of 5 or 6 is reduced compared to those that score 0, 1, 2, 3, or 4. In other disclosed embodiments, neurological impairment is assessed using the modified Rankin Scale and as a result of treatment the proportion of treated subjects with a modified Rankin Scale score of 4, 5 or 6 is reduced compared to those that score 0, 1, 2, or 3.

According to certain embodiments, the disclosure provides a Factor Xa (FXa) variant for use in reducing perihemorrhagic edema (PHE) of a subject after the subject has suffered ICH by administering to the subject a therapeutically effective amount of a Factor Xa (FXa) variant including at least one substitution mutation at the amino acid position corresponding to 235 or 236 in SEQ ID NO:1. In some disclosed embodiments, the substitution at the position corresponding to position 235 can be with Thr, Leu, Phe, Asp or Gly. In other disclosed embodiments, the substitution at the position corresponding to position 236 can be with Leu, Ala, or Gly. According to certain disclosed embodiments, treatment reduces the average volume of PHE in subjects with ICH by at least about 5%, 10%, 15%, 20%, 25%, 30%, 35%, 40%, 45%, 50%, 55%, 60%, 65%, 70%, 75%, 80%, 85%, 90%, or 95% compared to untreated controls. In other disclosed embodiments, treatment reduces the average increase in intracranial pressure caused by PHE in subjects with ICH by at least about 5%, 10%, 15%, 20%, 25%, 30%, 35%, 40%, 45%, 50%, 55%, 60%, 65%, 70%, 75%, 80%, 85%, 90%, or 95% compared to untreated controls.

According to certain disclosed embodiments, the disclosure provides a Factor Xa (FXa) variant for use in reducing or preventing hematoma volume expansion in a subject after the subject has suffered ICH by administering to the subject a therapeutically effective amount of a Factor Xa (FXa) variant including at least one substitution mutation at the amino acid position corresponding to 235 or 236 in SEQ ID NO:1. In some disclosed embodiments, the substitution at the position corresponding to position 235 can be with Thr, Leu, Phe, Asp or Gly. In other disclosed embodiments, the substitution at the position corresponding to position 236 can be with Leu, Ala, or Gly. According to certain disclosed embodiments, treatment reduces the average hematoma volume expansion in subjects with ICH by at least about 5%, 10%, 15%, 20%, 25%, 30%, 35%, 40%, 45%, 50%, 55%, 60%, 65%, 70%, 75%, 80%, 85%, 90%, or 95% compared to untreated controls. In other disclosed embodiments, treatment reduces the average hematoma volume expansion in subjects by at least about 1 ml, 1.5 ml, 2 ml, 2.5 ml, 3 ml, 3.5 ml, 4 ml, 4.5 ml, 5 ml, 5.5 ml, 6 ml, 6.5 ml, 7 ml, 7.5 ml, 8 ml, 8.5 ml, 9 ml, 9.5 ml, 10 ml, 10.5 ml, 11 ml, 11.5 ml, 12 ml, 12.5 ml, 13 ml, 13.5 ml, 14 ml, 14.5 ml, 15 ml, 16 ml, 17 ml, 18 ml, 19 ml, 20 ml, 22 ml, 24 ml, 26 ml, 28 ml, 30 ml, 35 ml, 40 ml, 45 ml, or 50 ml, compared to untreated controls. In other disclosed embodiments, treatment results in a reduction in the proportion of subjects in whom hematoma volume has expanded 3 ml or more, 6 ml or more, or 12.5 ml or more by at least about 5%, 10%, 15%, 20%, 25%, 30%, 35%, 40%, 45%, 50%, 55%, 60%, 65%, 70%, 75%, 80%, 85%, 90%, or 95%, compared to untreated controls. In other disclosed embodiments, treatment results in a reduction in the proportion of subjects in whom hematoma volume has expanded by 15%, 20%, 25%, 30%, or 33% or more by at least about 5%, 10%, 15%, 20%, 25%, 30%, 35%, 40%, 45%, 50%, 55%, 60%, 65%, 70%, 75%, 80%, 85%, 90%, or 95%, compared to untreated controls. In other disclosed embodiments, treatment results in a reduction in the proportion of subjects having a spot sign score of 4, 3, 2, or 1 after treatment by at least about 5%, 10%, 15%, 20%, 25%, 30%, 35%, 40%, 45%, 50%, 55%, 60%, 65%, 70%, 75%, 80%, 85%, 90%, or 95%, compared to untreated controls. In yet other disclosed embodiments, the spot sign score is determined 30 min, 45 min, 60 min, 75 min, 90 min, 2 hrs, 2.5 hrs, 3 hrs, 3.5 hrs, 4 hrs, 5 hrs, 6 hrs, 7 hrs, 8 hrs, 9 hrs, 10 hrs, 11 hrs, 12 hrs, 18 hrs, 24 hrs, 30 hrs, 48 hrs, 3 days, 4 days, 5 days, 6 days, 7 days, 8 days, 9 days, 10 days, 11 days, 12 days, 13 days, 14 days, 15 days, 18 days, 3 weeks, or 4 weeks after first administration of FXa variant.

According to other disclosed embodiments of the disclosure there are provided Factor Xa (FXa) variants including at least one substitution mutation at the amino acid position corresponding to 235 and/or 236 in SEQ ID NO:1 for use in treating intracerebral hemorrhage (ICH) in a subject, improving the brain function of a subject with ICH, for reducing neurological impairment of a subject with ICH, for reducing perihemorrhagic edema (PHE) in a subject with ICH or for reducing or preventing hematoma volume expansion in a subject with ICH. In some disclosed embodiments, the substitution at the position corresponding to position 235 can be with Thr, Leu, Phe, Asp or Gly. In some other disclosed embodiments, the substitution at the position corresponding to position 236 can be with Leu, Ala, or Gly.

And, according to yet other disclosed embodiments, any of the previous disclosed embodiments can be further characterized in that the intracerebral hemorrhage occurs in the brain's cerebrum, frontal lobe, parietal lobe, occipital lobe, temporal lobe, cerebellum, brain stem, midbrain, pons, medulla, pituitary gland, hypothalamus, thalamus, hippocampus, amygdala, claustrum, or basal ganglia.

### BRIEF DESCRIPTION OF THE DRAWINGS

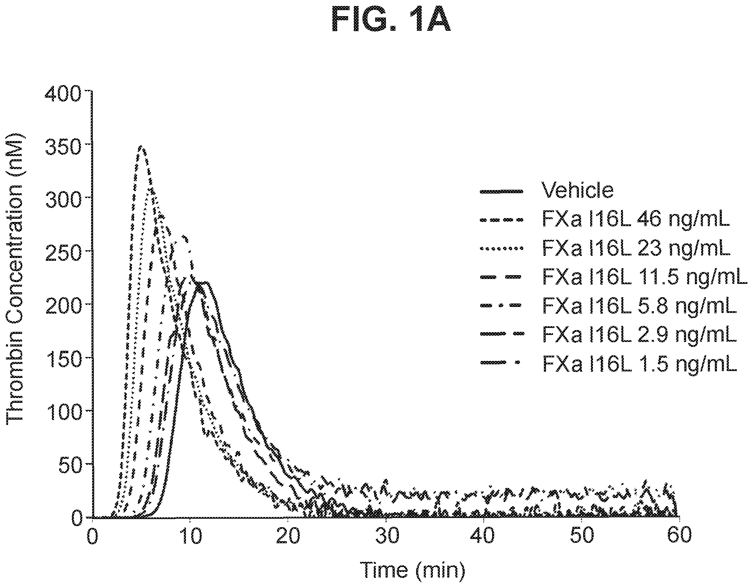

Figure 1A shows the results of a thrombin generation assay (TGA) testing the effect of adding different concentrations of activated Factor X variant FXa^{I16L} on the quantity of thrombin produced after coagulation was initiated in samples of normal human plasma. Over the 60-minute time course of the experiment, the addition of FXa^{I16L} resulted in a dose dependent increase in thrombin generation compared to plasma treated with vehicle only.

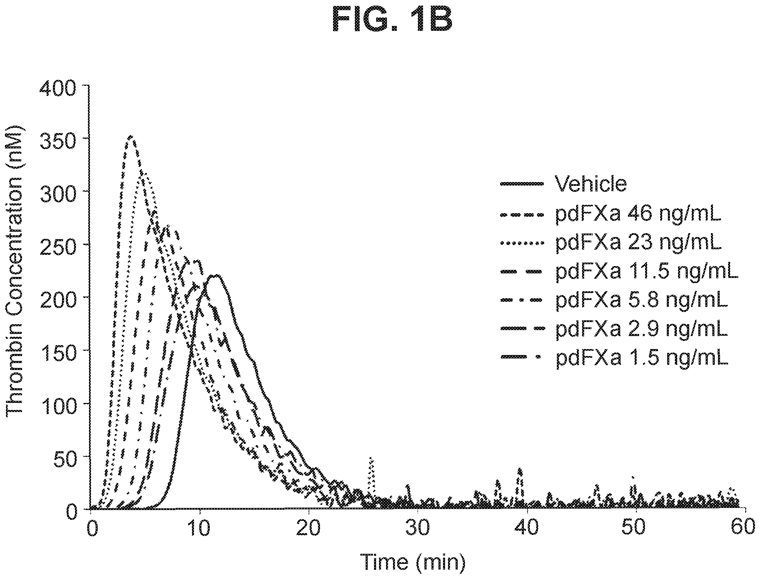

Figure 1B shows the results of a thrombin generation assay (TGA) testing the effect of adding different concentrations of plasma-derived activated Factor X pdFXa on the quantity of thrombin produced after coagulation was initiated in samples of normal human plasma. Over the 60-minute time course of the experiment, the addition of pdFXa resulted in a dose dependent increase in thrombin generation compared to plasma treated with vehicle only.

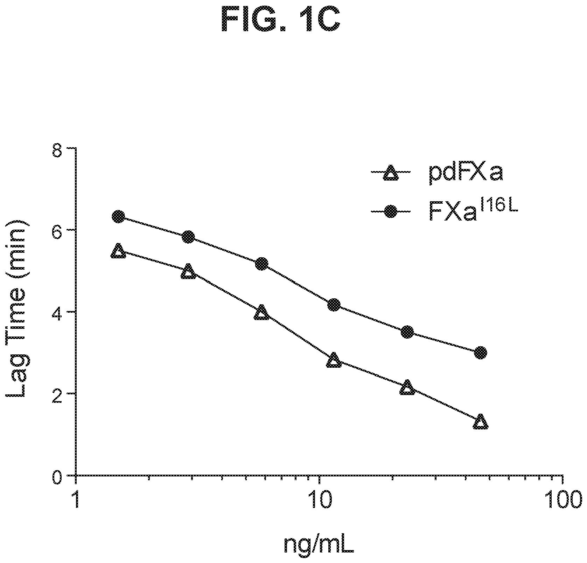

Figure 1C shows the lag time for the initiation phase of coagulation calculated from the data from the experiments described in Figure 1A and Figure 1B. Compared to human plasma treated with vehicle only, both pdFXa and FXa^{I16L} shortened lag time, although lag time for FXa^{I16L} treated plasma was longer than plasma treated with pdFXa.

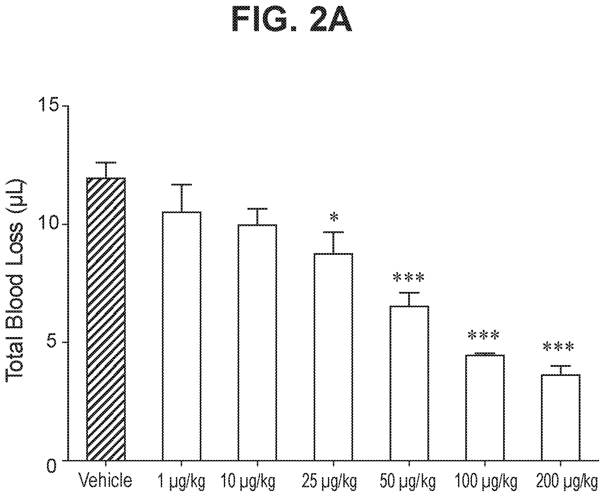

Figure 2A shows the effect of pretreatment with increasing concentrations of FXa^{I16L} on blood loss from non-hemophilic mice after tail transection. FXa^{I16L} treatment before tail transection caused a dose-dependent decrease in total blood loss compared to mice treated with vehicle only.

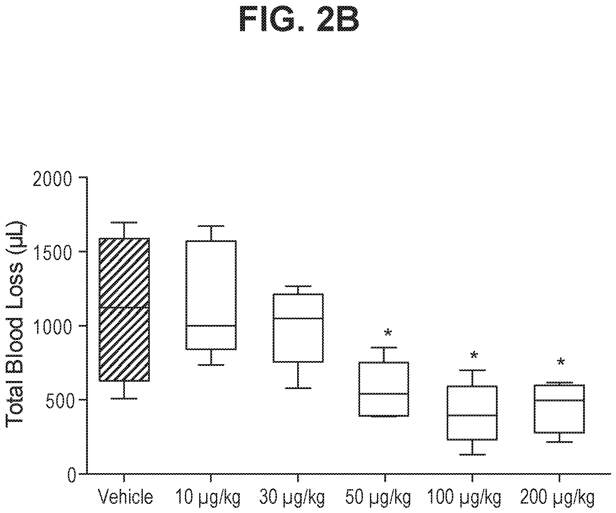

Figure 2B shows the effect of pretreatment with increasing concentrations of FXa^{I16L} on blood loss from non-hemophilic rats after tail transection. FXa^{I16L} treatment before tail transection caused a dose-dependent decrease in total blood loss compared to rats treated with vehicle only.

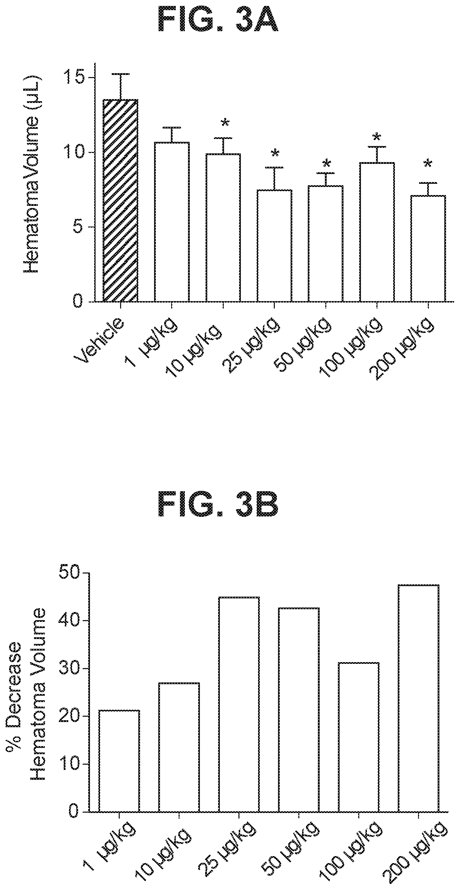

Figure 3A shows the effect of treatment with increasing concentrations of FXa^{I16L} on hematoma volume in mice experimentally manipulated to produce intracerebral bleeding as an animal model for ICH. FXa^{I16L} treatment after experimentally induced bleeding into the brain tissue caused a dose-dependent decrease in hematoma volume compared to mice treated with vehicle only.
Figure 3B shows the percent decrease in hematoma volume compared to vehicle in relation to dose based on the data in Figure 3A.

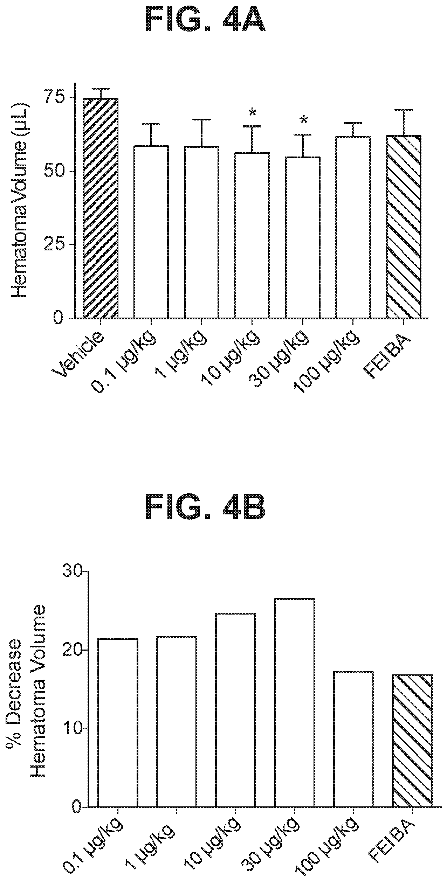

Figure 4A shows the effect of treatment with increasing concentrations of FXa^{I16L} on hematoma volume in rats experimentally manipulated to produce intracerebral bleeding as an animal model for ICH. FXa^{I16L} treatment after experimentally induced bleeding into the brain tissue caused a dose-dependent decrease in hematoma volume compared to rats treated with vehicle only.
Figure 4B shows the percent decrease in hematoma volume compared to vehicle in relation to dose based on the data in Figure 4A.

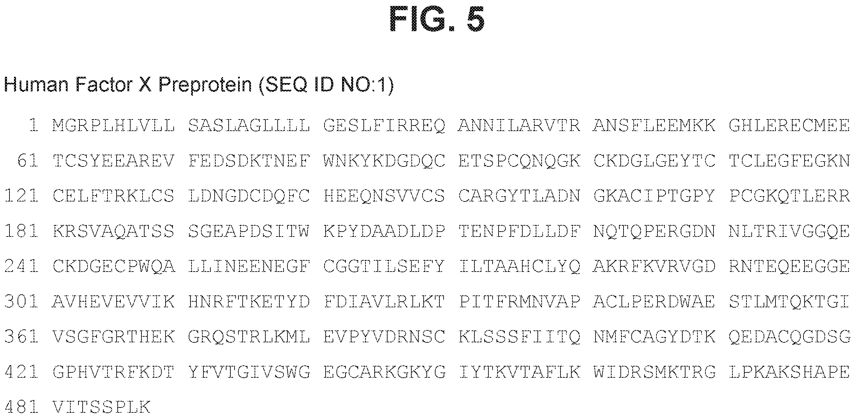

Figure 5 is the amino acid sequence of wild-type human Factor X preprotein (SEQ ID NO:1). The signal peptide corresponds to amino acids 1-23. The propeptide corresponds to amino acids 24-40. The mature light chain of activated Factor X (FXa) corresponds to amino acids 41-179. The mature heavy chain of activated FXa (after removal of the activation peptide) corresponds to amino acids 235-488. The activation peptide (AP) corresponds to amino acids 183-234.

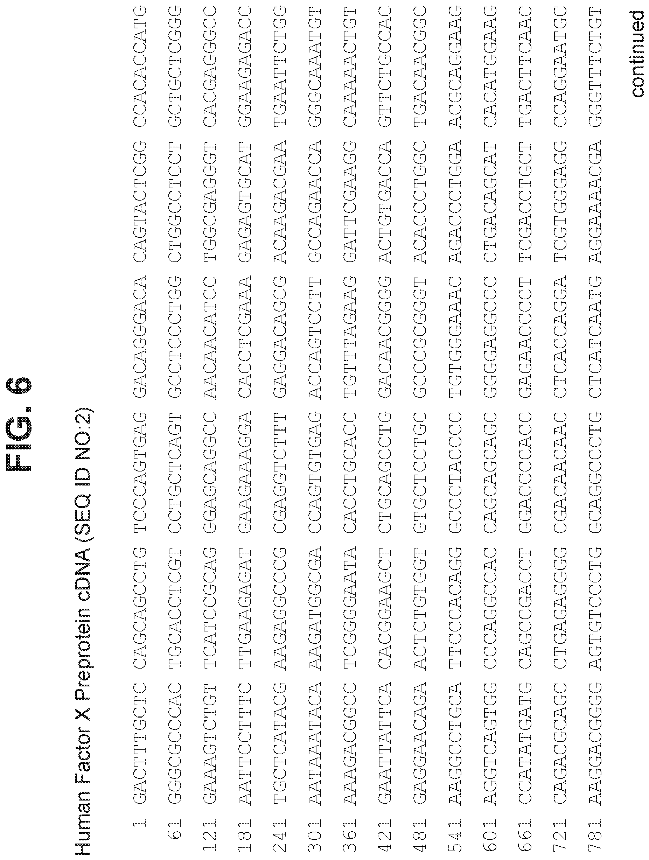

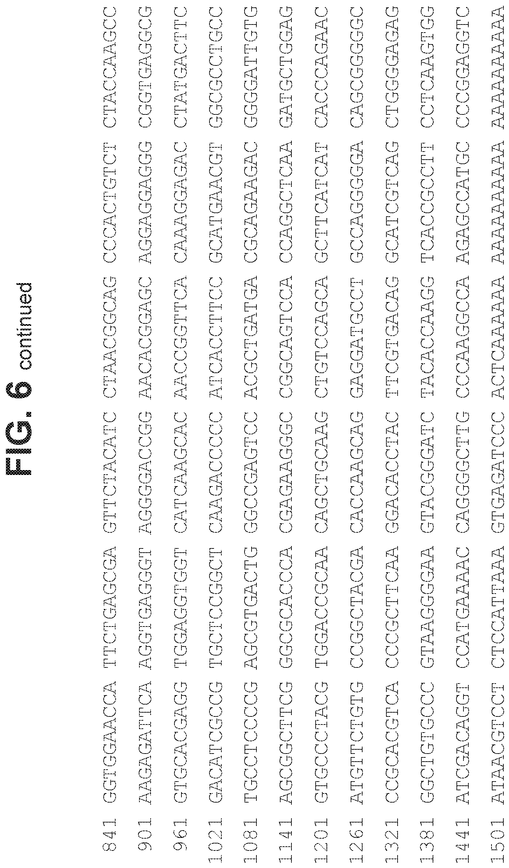

Figure 6 is the nucleotide sequence of the cDNA encoding wild-type human Factor X preprotein (SEQ ID NO:2). The coding sequence corresponds to nucleotides 58 to 1524.

### DETAILED DESCRIPTION

It was previously demonstrated that a variant of the clotting Factor Xa having zymogenic properties is effective to inhibit uncontrolled bleeding in hemophilic animals. It was not known or predicted, however, whether this variant could effectively stop uncontrolled bleeding in animals with intact coagulation systems. Applicants have demonstrated that this variant is an effective procoagulant in non-hemophilic animals and can also reduce hematoma expansion in non-hemophilic animal models of intracerebral hemorrhage. Based on these new studies, FXa variants of the disclosure, and compositions comprising such variants, are expected to be useful for use in treating and preventing ICH in non-hemophilic subjects, including humans and non-human animals. Treating an ICH subject with a FXa variant can improve the subject's quality of life and neurological functioning and reduce the severity of symptoms and impairment caused by ICH compared to untreated controls. Factor Xa (FXa) variants for use in treating and preventing ICH using the FXa variants and compositions of the present disclosure are described in further detail below.

### FACTOR XA AND VARIANTS THEREOF

Coagulation factor X (FX) is a zymogen which, upon activation by the intrinsic factor IX/factor VIII or extrinsic pathway (tissue factor/factor VIIa), becomes FXa, which is the protease moiety of prothrombinase. Following proteolytic cleavage of the Arg-Ile scissile bond, releasing an activation peptide (AP), a series of well defined structural changes in the zymogen drives the activation process to the mature active serine protease (See Toso et al., (2008) J. Biol. Chem. 283, 18627-18635; Bunce et al., (2011) Blood 117, 290-298; and Ivanciu et al., (2011) Nat. Biotechnol. 29, 1028-1033). The mature FXa has a light chain and a heavy chain that contains the catalytic domain. The mature FXa becomes an active serine protease upon formation of the prothrombinase complex, which includes binding of an activated cofactor, Factor Va (FVa).

Variant forms of FX have been developed that upon activation cleavage yield a zymogen-like FXa variant. That is, once cleaved, the resulting FXa variant has poor active site function and is more resistant to inactivation by circulating inhibitors (i.e. antithrombin III and TFPI). The FXa variants, thus, have longer half-lives in plasma than wild-type FXa. The FXa variant binds FVa, lipid membrane and calcium to form a fully active prothrombinase complex that efficiently activates prothrombin.

The zymogen-like variants of FXa circulate in the zymogen-like conformation and do not seem to be thrombogenic (See Toso et al., (2008) J. Biol. Chem. 283, 18627-18635 and Ivanciu et al., (2011) Nat. Biotechnol. 29, 1028-1033). Examples of such FXa variants are described in International patent publication WO2007/059513.

The enzymes of coagulation are trypsin-like enzymes that belong to the S1 peptidase family of proteases that bear a chymotrypsin-like fold. The coagulation proteases contain catalytic domains that are highly homologous to each other and to the ancestral serine proteases of digestion. The structural homology/identity is so great (>70%) that residues in the catalytic domains of the coagulation enzymes (including Factor Xa) are numbered according to the corresponding residues in chymotrypsinogen. (Chymotrypsin numbering system; see Bajaj and Birktoft, Methods Enzymol. 1993; 222:96-128, Table 2, and Bode W, Mayr I, Bauman Y, et al. The refined 1.9 Å crystal structure of human alpha-thrombin: interaction with D-Phe-Pro-Arg chloromethylketone and significance of the Tyr-Pro-Trp insertion segment. EMBO J 1989;8(11):3467-3475). Accordingly, amino acids may be referred to herein according to the chymotrypsin numbering system, which is well-known to those of skill in the art.

According to the disclosure, an FXa variant may be an FXa protein comprising an amino acid substitution that makes the variant more zymogen-like compared to a wild-type FXa protein *in vivo* or *in vitro.* FXa variants of the disclosure substantially regain wild-type FXa activity upon formation of prothrombinase. Examples of FXa variants that are useful in medical uses of the disclosure are variants comprising a modification selected from the group consisting of: a) Ile at position 16 is Thr, Leu, Phe, Asp or Gly and b) Val at position 17 is Leu, Ala, or Gly, according to the chymotrypsin numbering system. Amino acids 16 and 17 in the chymotrypsin numbering system occur at amino acids 235 and 236, respectively, of SEQ ID NO:1 (human Factor X preproprotein). In certain disclosed embodiments, FXa variants are FXa^{I16L} and FXa^{I16T} (the nomenclature used herein for the FXa variants recites the original amino acid at the numbered position according to the chymotrypsin numbering system followed by the substituted amino acid). The FXa variants can be variants of any mammalian FXa. Of particular interest, however, are FXa variants of human FXa.

In certain disclosed embodiments, the FX variant that is activated into a variant FXa of the disclosure may be further modified by inserting a non-native intracellular proteolytic cleavage site. In a non-limiting example, to express "activated" zymogen-like FXa variants in mammalian cells, a non-native intracellular proteolytic cleavage site can be inserted between the Arg at position 234 of SEQ ID NO:1 (position 15 in the chymotrypsin numbering system) and the amino acid at the position corresponding to position 235 of SEQ ID NO:1 (position 16 in the chymotrypsin numbering system) in the variant FX zymogen. In certain disclosed embodiments, the non-native intracellular protease cleavage site is Arg-Lys-Arg or Arg-Lys-Arg-Arg-Lys-Arg (SEQ ID NO:3). These cleavage sites are efficiently recognized by proteases (PACE/furin-like enzymes) within the cell and are removed. This cleavage may result in a processed variant FXa in which the mature heavy chain of the molecule now begins at the amino acid at the position corresponding to position 235 of SEQ ID NO:1 (position 16 in the chymotrypsin numbering system). Introduction of this cleavage site at said position allows for the intracellular conversion of FX to FXa.

In certain disclosed embodiments the entire amino acid sequence of the FX variant activation peptide (AP) (i.e., amino acids 183-234 of SEQ ID NO:1) is replaced with a non-native intracellular protease cleavage site. According to certain disclosed embodiments, the non-native intracellular protease cleavage site is Arg-Lys-Arg or Arg-Lys-Arg-Arg-Lys-Arg (SEQ ID NO:3). As explained above, this modification allows for intracellular cleavage of the FX variant expressed by cells. The intracellular cleavage converts FX variant to activated zymogen-like FXa variant which is then secreted by cells for subsequent purification. This approach obviates the need for extracellular cleavage that would otherwise be required to activate the variant clotting factor, for example, after isolating the protein or just before blood clotting.

In certain disclosed embodiments, FXa variants of the disclosure are derived from FX variant preproteins comprising native wild-type human signal sequence and/or propeptide sequence. In other disclosed embodiments, FX signal sequences and/or propeptide from non-human species can be used in place of the corresponding native amino acid sequences. And in yet other disclosed embodiments, signal sequence and/or propeptide sequence from other human or non-human secreted proteins can be used in place of the corresponding native amino acid sequences.

In an exemplary disclosed embodiment, a FXa variant comprises amino acids 41-179 and amino acids 235-488 of SEQ ID NO:1, wherein the amino acid at position 235 (isoleucine in the wild-type sequence) is substituted with a different amino acid selected from the group consisting of threonine (Thr), leucine (Leu), phenylalanine (Phe), aspartic acid (Asp), or glycine (Gly). These substitutions can respectively be written using the nomenclature I235T, I235L, I235F, I235D and I235G, where the first letter is the single letter code for isoleucine and the last letter is the single letter code for the amino acid being substituted into the wild-type sequence. Because position 235 of SEQ ID NO:1 is equivalent to position 16 in the chymotrypsin numbering system, the same substitutions can be written 116T, 116L, 116F, 116D and 116G. In a disclosed embodiment, a FXa variant comprises amino acids 41-179 and amino acids 235-488 of SEQ ID NO:1, wherein the amino acid at position 235 is substituted with Thr (i.e., I235T or I16T). In a disclosed embodiment, a FXa variant comprises amino acids 41-179 and amino acids 235-488 of SEQ ID NO:1, wherein the amino acid at position 235 is substituted with Leu (i.e., I235L or I16L). In a disclosed embodiment, a FXa variant comprises amino acids 41-179 and amino acids 235-488 of SEQ ID NO:1, wherein the amino acid at position 235 is substituted with Phe (i.e., I235F or I16F). In a disclosed embodiment, a FXa variant comprises amino acids 41-179 and amino acids 235-488 of SEQ ID NO:1, wherein the amino acid at position 235 is substituted with Asp (i.e., I235D or I16D). In a disclosed embodiment, a FXa variant comprises amino acids 41-179 and amino acids 235-488 of SEQ ID NO:1, wherein the amino acid at position 235 is substituted with Gly (i.e., I235G or I16G).

According to another exemplary disclosed embodiment, a FXa variant comprises amino acids 41-179 and amino acids 235-488 of SEQ ID NO:1, wherein the amino acid at position 236 (valine in the wild-type sequence) is substituted with a different amino acid selected from the group consisting of leucine (Leu), alanine (Ala), or glycine (Gly). These substitutions can respectively be written using the nomenclature V236L, V236A, and V236G, where the first letter is the single letter code for valine and the last letter is the single letter code for the amino acid being substituted into the wild-type sequence. Because position 236 of SEQ ID NO:1 is equivalent to position 17 in the chymotrypsin numbering system, the same substitutions can be written V17L, V17A, and V17G. In a disclosed embodiment, a FXa variant comprises amino acids 41-179 and amino acids 235-488 of SEQ ID NO:1, wherein the amino acid at position 236 is substituted with Leu (i.e., V236L or V17L). In a disclosed embodiment, a FXa variant comprises amino acids 41-179 and amino acids 235-488 of SEQ ID NO:1, wherein the amino acid at position 236 is substituted with Ala (i.e., V236A or V17A). In a disclosed embodiment, a FXa variant comprises amino acids 41-179 and amino acids 235-488 of SEQ ID NO:1, wherein the amino acid at position 236 is substituted with Gly (i.e., V236G or V17G).

In other disclosed embodiments, FXa variants of the disclosure, including those specific variants described in the preceding paragraphs, can include various isoforms of the light and/or mature heavy chain of the protein. Non-limiting exemplary isoforms of the FXa variant mature heavy chain include the alpha and beta versions of the mature heavy chain. Jesty et al., J Biol Chem. 1975 Jun 25;250(12):4497-504. Compositions of the disclosure can include FXa variant proteins in which one or the other or both alpha and beta mature heavy chain isoforms are represented.

According to yet other disclosed embodiments, isoforms of FXa variant proteins, including those specific variants described in the preceding paragraphs, can include isoforms in which a variable number of amino acids (for example, 1, 2, 3, 4, 5, 6, or more amino acids) are either missing from or added to the carboxy terminus of the light chain and/or mature heavy chains of the protein.

According to certain disclosed embodiments, FXa variants of the disclosure include proteins with a certain minimal degree of homology or sequence identity compared to the amino acid sequence of wild-type FXa in SEQ ID NO:1. Thus, for example, FXa variants include proteins that contain a light and mature heavy chain that are at least 60%, 70%, 80%, 85%, 90%, 95%, 98%, or 99% homologous or identical in sequence with the wild-type FXa light and mature heavy chains in SEQ ID NO:1, wherein such FXa variants also include a substitution at the amino acid position corresponding to position 235 of SEQ ID NO:1 with Thr, Leu, Phe, Asp, or Gly, or a substitution at the amino acid position corresponding to position 236 of SEQ ID NO:1 with Leu, Ala, or Gly, and further wherein such FXa variants are zymogenic until incorporated into prothrombinase complex. In the amino acid sequence of SEQ ID NO:1, the wild-type FXa light chain sequence corresponds to amino acids 41 to 179 and the wild-type FXa mature heavy chain sequence corresponds to amino acids 235 to 488. Percentage amino acid sequence homology or identity can readily be determined using software such as Protein BLAST available at the website of the National Center for Biotechnology Information (http://blast.ncbi.nlm.nih.gov/Blast.cgi).

According to other non-limiting disclosed embodiments, FXa variants of the disclosure can also include FXa variants containing one or more post-translational modifications including, without limitation, one or more O-linked or N-linked carbohydrate groups or a variable number of gamma-carboxyglutamic acid (Gla) residues. FXa variants of the disclosure can further include chemically modified FXa variant proteins. Other FXa variants useful in the medical use of the disclosure are also possible.

As used herein, the term FXa^{I16x} refers to a variant of activated Factor X wherein the amino acid corresponding to position 235 in SEQ ID NO:1 (corresponding to position 16 in the chymotrypsin numbering system) is changed from the amino acid in the wild-type sequence (isoleucine) to a different amino acid denoted "x". In some non-limiting exemplary disclosed embodiments, amino acid "x" can be threonine (Thr or T), leucine (Leu or L), phenylalanine (Phe or F), aspartic acid (Asp or D), or glycine (Gly or G).

As used herein, the term FXa^{V17y} refers to a variant of activated Factor X wherein the amino acid corresponding to position 236 in SEQ ID NO:1 (corresponding to position 17 in the chymotrypsin numbering system) is changed from the amino acid in the wild-type sequence (valine) to a different amino acid denoted "y". In some non-limiting exemplary disclosed embodiments, amino acid "y" can be leucine (Leu or L), alanine (Ala or A), or glycine (Gly or G).

The terms FXa^{I16x} and FXa^{V17y} are not limited by the protein sequence set forth in SEQ ID NO:1. Rather these terms additionally include the variety of isoforms and homologous proteins described herein with the specified substitution mutations at positions 16 or 17 in the chymotrypsin numbering system that behave as zymogens until incorporated into prothrombinase complex.

An FXa variant of the disclosure may be produced by any technique for expressing a protein.

An "isolated protein," "isolated polypeptide" or "isolated variant" is a protein, polypeptide or variant that by virtue of its origin or source of derivation (1) is not associated with naturally associated components that accompany it in its native state, (2) is free of other proteins from the same species, (3) is expressed by a cell from a different species, or (4) does not occur in nature. Thus, a polypeptide that is chemically synthesized or synthesized in a cellular system different from the cell from which it naturally originates will be "isolated" from its naturally associated components. A protein may also be rendered substantially free of naturally-associated components by isolation, using protein purification techniques well known in the art.

A protein or polypeptide is "substantially pure," "substantially homogeneous," or "substantially purified" when at least about 60 to 75% of a sample exhibits a single species of polypeptide. The polypeptide or protein may be monomeric or multimeric. A substantially pure polypeptide or protein will typically comprise about 50%, 60%, 70%, 80% or 90% W/W of a protein sample, more usually about 95%, and may be over 99% pure. Protein purity or homogeneity may be indicated by a number of means well known in the art, such as polyacrylamide gel electrophoresis of a protein sample, followed by visualizing a single polypeptide band upon staining the gel with a stain well known in the art. For certain purposes, higher resolution may be provided by using HPLC or other means well known in the art for purification.

### INTRACEREBRAL HEMORRHAGE

Intracerebral hemorrhage (ICH) is a form of stroke resulting from the rupture of a diseased or damaged blood vessel in the brain causing bleeding into the brain tissue (parenchyma). Brain damage and consequent neurological impairment or death can result through different mechanisms. For example, it is thought that blood leakage reduces oxygenation to the brain cells fed by the affected blood vessels. It is also believed that pooling blood can cause edema, and that the hematoma which eventually forms can also exert pressure on surrounding brain tissue. Both edema and hematoma can increase intracranial pressure. Other mechanisms of brain damage caused by ICH are possible. The particular mechanism or mechanisms responsible for causing brain damage after ICH generally or in specific cases are not intended to limit the disclosed embodiments of the disclosure in any way.

ICH can occur in any part of the brain. Exemplary regions of the brain in which ICH can occur, or that can be affected by ICH occurring in a different part of the brain, include but are not limited to cerebrum (including frontal lobe, parietal lobe, occipital lobe, temporal lobe), cerebellum, brain stem (including midbrain, pons, medulla), pituitary gland, hypothalamus, thalamus, hippocampus, amygdala, claustrum, or basal ganglia.

ICH can occur as a result of bleeding from an artery in the brain. Exemplary arteries of the brain include but are not limited to anterior cerebral artery (ACA), middle cerebral artery (MCA), posterior cerebral artery (PCA), ophthalmic artery, arteries of the circle of Willis, posterior inferior cerebellar arteries (PICA), or lenticulostriate arteries.

Although certain symptoms indicative of increased intracranial pressure, such as headache, nausea, vomiting, and loss of consciousness, may suggest the existence of ICH, definitive diagnosis requires brain imaging. Brain imaging (also called neuroimaging) techniques familiar to those of ordinary skill in the art include computed tomography (CT) scans, angiography, CT angiography, magnetic resonance imaging (MRI) and MR angiography. Other techniques for the definitive diagnosis of ICH will be familiar to those of ordinary skill in the art.

ICH is less common than ischemic stroke, which is caused by blockage of blood vessels in the brain by a thrombus, but comprises about 12 per cent of all strokes. In contrast to ischemic strokes, ICH is more likely to significantly worsen in the first 24 hours after bleeding starts, particularly when the hemorrhage is accompanied by expansion of the hematoma. As will be discussed further below, hematoma expansion is associated with worse patient outcome.

### FACTOR XA (FXA) VARIANTS FOR USE IN TREATING INTRACEREBRAL HEMORRHAGE (ICH)

The present disclosure provides a Factor Xa (FXa) variant for use in for treating or preventing ICH by administering to subjects in need of treatment for ICH a therapeutically effective amount of a FXa variant having zymogenic properties, for example, the FXa variant FXa^{I16L}.

As used herein, the term "subject" refers to any animal with a brain that is capable of suffering intracerebral hemorrhage and a coagulation system that can be activated using FXa variants of the disclosure. In some disclosed embodiments, the animal is a mammal, for example, humans and non-human primates (e.g., apes, monkeys, baboons, chimpanzees), or other mammals, such as rodents (e.g., mice, rats), bovines, horses, cats, dogs, pigs, rabbits, goats, deer, sheep, ferrets, gerbils, guinea pigs, hamsters, and bats. Other animals are possible according to the knowledge of those of ordinary skill in the art.

In some disclosed embodiments, subjects are non-hemophilic mammals, for example non-hemophilic humans. In other disclosed embodiments, subjects have hemophilia, which can include hemophilia A, hemophilia B or other types of hemophilia.

As used herein, the phrase "therapeutically effective amount" refers to an amount of FXa variant (e.g., FXa^{I16L}) which, when administered to a subject with ICH, results in a reduced likelihood that the subject will die as a result of ICH, results in reduced morbidity caused by ICH, results in reduced neurological impairment caused by ICH, or results in an improvement in the functioning, quality of life or condition of the subject after ICH. A therapeutically effective amount of a FXa variant can additionally prevent or reduce the extent to which the hematoma caused by ICH expands in volume after bleeding begins or ICH is diagnosed, prevent expansion or reduce the volume of peri-hemorrhagic edema caused by ICH, or prevent or reduce the increase of intracranial pressure that can attend ICH. A therapeutically effective amount of a FXa variant can additionally delay the onset, or mitigate, lessen or attenuate the severity, of any of the physical signs, symptoms, physiological effects, or neurological impairment caused by ICH. A therapeutically effective amount of a FXa variant may vary according to factors such as the properties of the particular FXa variant to be used, the disease state, age, sex, and weight of the subject to be treated, and the ability of the FXa variant to elicit a desired response in the subject. A therapeutically effective amount is also one in which any toxic or detrimental effects of the FXa variant are outweighed by the therapeutically beneficial effects.

The phrase "prophylactically effective amount" refers to an amount of FXa variant which, when administered to a subject having one or more risk factors for ICH, reduces the likelihood that the subject will suffer ICH or reduces the incidence of ICH in populations of subjects with similar risk profiles.

Efficacy and efficacious amounts of FXa variant can be determined based on observations of individual ICH subjects administered FXa variant. Efficacy and efficacious amounts of FXa variant can also be determined by conducting controlled clinical studies in defined populations of ICH subjects according to the knowledge of those of ordinary skill in the art. Based on the results of such clinical studies, care providers are able to predict ahead of time what is likely to be an effective amount of FXa variant before it is administered to a subject newly experiencing and in need of treatment for ICH.

Although desirable, complete prevention or reversal of the physical signs or symptoms of ICH (including, for example, hematoma expansion), or the neurological impairment caused by ICH, are not required for efficacy to exist. Rather, as indicated above, an effective amount of FXa variant need only improve an ICH subject's condition compared to what that subject's condition would likely have been absent treatment with FXa variant.

### FACTOR XA (FXA) VARIANTS FOR USE IN REDUCING DEATH RATE DUE TO ICH

It has been reported that 30-day fatality rates caused by ICH range from 40% to 50% with about half of deaths occurring within 2 days of onset. ML Flaherty et al, "The epidemiology of intracerebral hemorrhage," in Intracerebral Hemorrhage, ed. JR Carhuapoma, et al (Cambridge: Cambridge University Press, 2010).

According to certain disclosed embodiments, administering an effective amount of FXa variant (e.g., FXa^{I16L}) to subjects with ICH can reduce the incidence of death (death rate) caused by ICH. This could be demonstrated, for example, by comparing the relative ability of FXa variant to reduce the death rate in a defined population of ICH subjects compared to an untreated control population. In some disclosed embodiments, treating subjects with ICH reduces death rate caused by ICH from about 40% - 50% to about 45%, about 40%, about 35%, about 30%, about 25%, about 20%, about 15%, about 10% or less. In some other disclosed embodiments, treatment with FXa variant reduces the death rate caused by ICH by about 5%, 10%, 15%, 20%, 25%, 30%, 35%, 40%, 45%, 50%, 55%, 60%, 65%, 70%, 75%, 80%, 85%, 90% or more compared to the death rate of ICH subjects not treated with the FXa variant. Death rate can be determined at any time after ICH onset. Non-limiting exemplary times after onset include 1 day, 2 days, 3 days, 4 days, 5 days, 6 days, 7 days, 10 days, 14 days, 15 days, 20 days, 21 days, 25 days, 28 days, 30 days, or more.

### FACTOR XA (FXA) VARIANTS FOR USE IN IMPROVING BRAIN FUNCTION AFTER ICH

In a disclosed embodiment of the medical uses of the disclosure, administration of an effective amount of FXa variant (e.g., FXa^{I16L}) improves brain function in an ICH subject.

In one disclosed embodiment, improved brain function is associated with reducing elevated intracranial pressure (ICP) caused by ICH below certain maximal levels. For example, ICP can be reduced to levels equal or below 80 mm Hg, 70 mm Hg, 60 mm Hg, 50 mm Hg, 40 mm Hg, 30 mm Hg, 20 mm Hg, 10 mm Hg, or less. By reducing ICP below certain levels, the likelihood of the ICH subject dying is reduced. Methods for monitoring ICP are familiar to those of ordinary skill in the art. S.-B. Ko, et al., Stroke 42:3087-3092 (2011).

In another disclosed embodiment, improved brain function is associated with maintaining a certain minimal level of cerebral perfusion pressure (CPP). For example, CPP can be maintained at levels equal or exceeding 40 mm Hg, 50 mm Hg, 60 mm Hg, 70 mm Hg, 80 mm Hg, 90 mm Hg, 100 mm Hg, 110, mm Hg, 120 mm Hg, or higher. By maintaining CPP at sufficient levels, the likelihood of the ICH subject experiencing brain metabolic crisis or brain tissue hypoxia is reduced. Sufficiently high CPP is also associated with reduced ICH subject mortality. Methods for monitoring CPP are familiar to those of ordinary skill in the art. S.-B. Ko, et al., Stroke 42:3087-3092 (2011).

In yet another disclosed embodiment, improved brain function is associated with maintaining a certain minimal level of brain tissue oxygen tension (P_{bt}O₂). For example, P_{bt}O₂ can be maintained at levels equal or exceeding 6 mm Hg, 8 mm Hg, 10 mm Hg, 12 mm Hg, 14 mm Hg, 16 mm Hg, 18 mm Hg, 20, mm Hg, 22 mm Hg, 24 mm Hg, or higher. By maintaining P_{bt}O₂ at sufficient levels, the likelihood of the ICH subject experiencing brain metabolic crisis or brain tissue hypoxia is reduced. Sufficiently high P_{bt}O₂ is also associated with reduced ICH subject mortality. Methods for monitoring P_{bt}O₂ are familiar to those of ordinary skill in the art. S.-B. Ko, et al., Stroke 42:3087-3092 (2011).

In a further disclosed embodiment, improved brain function is associated with reducing the ratio of the concentrations of lactate to pyruvate (LAR) below certain levels. Higher ratios are indicative of anaerobic metabolism. For example, LAR can be reduced to levels equal or below 60, 50, 40, 30, 20, or less. By reducing LAR below certain levels, the likelihood of the ICH subject dying is reduced. Methods for monitoring brain lactate and pyruvate concentrations are familiar to those of ordinary skill in the art. S.-B. Ko, et al., Stroke 42:3087-3092 (2011).

In another disclosed embodiment, improved brain function is associated with reducing the ratio of the cerebrovascular pressure reactivity index (PRx), which is calculated as the moving correlation coefficient between mean intracranial pressure (ICP) and mean arterial blood pressure (MAP). C. Zweifel, et al., Neurosurg. Focus, 25(10):E2 (2008). For example, PRx can be reduced to levels equal or below 0.5, 0.4, 0.3, 0.2, 0.1, 0.0, -0.1, -0.2, -0.3, or less. By reducing PRx below certain levels, the likelihood of the ICH subject dying is reduced. Methods for monitoring ICH and MAP can calculating PRx are familiar to those of ordinary skill in the art. S.-B. Ko, et al., Stroke 42:3087-3092 (2011).

### FACTOR XA (FXA) VARIANTS FOR USE IN REDUCING NEUROLOGICAL IMPAIRMENT AFTER ICH

Apart from death, the most significant impact of ICH on subjects is neurological impairment, often severe, which can devastate the quality of life of the subject and the subject's family, as well as impose substantial societal costs. Among other things, neurological impairment can interfere or prevent an ICH subject from working or carrying on ordinary daily activities without assistance. By treating or preventing ICH in subjects using FXa variants of the disclosure, the degree of neurological impairment caused by ICH can be reduced compared to the extent of impairment that would occur in the absence of treatment in the same subject or in similar untreated control subjects. As a result, the ability of ICH subjects treated according to the medical use of the disclosure to work or function independently are improved.

Neurological impairment can be quantified using a neurological function assessment tool. Non-limiting examples of such assessment tools include the Glasgow Coma Score (GCS), the Glasgow Outcome Scale, extended version (GOS-E), the Barthel Index (BI), the NIH Stroke Scale (NIHSS), and the modified Rankin Scale (mRS). Other assessment tools can also be used according to the knowledge of those ordinarily skilled in the art, including for example, the EuroQol scale, the Revised Hamilton Rating Scale for Depression and others.

The Glasgow Coma Score is intended to describe the level of consciousness in a subject following ICH or other brain injury. The GCS is scored between 3 and 15, 3 being the worst and 15 the best. It is composed of three parameters, including Best Eye Response, Best Verbal Response and Best Motor Response. For Best Eye Response, no eye opening = 1; eye opening to pain = 2; eye opening to verbal command = 3 and eyes open spontaneously = 4. For Best Verbal Response, no verbal response = 1; incomprehensible sounds = 2; inappropriate words = 3; confused = 4 and orientated = 5. For Best Motor Response, no motor response = 1; extension to pain = 2; flexion to pain = 3; withdrawal from pain = 4; localising pain = 5 and obeys commands = 6. A GSC of 13 or higher correlates with a mild brain injury, 9 to 12 is a moderate injury and 8 or less a severe brain injury.

The Glasgow Outcome Scale (GOS) and its extended version (GOS-E) are intended to describe how well a subject has recovered after ICH or other brain injury. The GOS has five levels and associated scores, including death = 1; vegetative state (subject alive but unresponsive) = 2; severely disabled (subject is conscious but requires others for daily support due to disability) = 3; moderately disabled (subject is independent but disabled) = 4 and good recovery (subject has resumed most normal activities but may have minor residual problems) = 5.

In the Extended GOS, or GOS-E, the scoring system is expanded to eight levels in which Dead = 1; Vegetative State = 2; Lower Severe Disability = 3; Upper Severe Disability = 4; Lower Moderate Disability = 5; Upper Moderate Disability = 6; Lower Good Recovery = 7 and Upper Good Recovery = 8.

The Barthel Index (BI) is a rating scale for measuring activity limitations in subjects after ICH or other brain injury. Mahoney F. and Barthel D., Functional evaluation: the Barthel Index., Md Med J. 14:61-65 (1965). The scale consists of ten functional categories, including feeding ability, bathing ability, grooming ability, dressing ability, bowel control, bladder control, toilet use, ability to transfer between bed to chair and back, mobility, and ability to use stairs. Subjects are scored for the amount of assistance required to accomplish the activity specified in each category. The category scores are then summed to yield an overall activity score. Among different versions of the BI scale, the categories are consistent, but the numeric values assigned to degree of dependency vary so that minimum and maximum summed scores vary with the version used. For example, in some versions, scores range from 0 to 20, whereas in other versions, scores range from 0 to 100, but in all versions the higher the score, the greater the ability of the subject to function independently.

The NIH Stroke Scale (NIHSS) is a 15-item neurologic examination stroke scale used to evaluate the effect of ICH, stroke or other brain injury on the levels of consciousness, language, neglect, visual-field loss, extraocular movement, motor strength, ataxia, dysarthria, and sensory loss. A trained observer rates the subject's ability to answer questions and perform activities. Ratings for each item are scored with 3 to 5 grades with 0 as normal. A score of 0 indicates no stroke symptoms, score of 1-4 indicates minor stroke, score of 5-15 indicates moderate stroke, score of 16-20 indicates moderate to severe stroke, and score of 21-42 indicates severe stroke.

The modified Rankin Scale (mRS) measures the degree of disability or dependence in the daily activities of subjects after ICH, stroke or other brain injury. Scores range from 0 to 6, where 0 indicates no symptoms of neurological deficit; 1 indicates no significant disability despite presence of some symptoms; 2 indicates slight disability (e.g., able to function independently, but unable to carry out all previous activities); 3 indicates moderate disability (e.g., requires some help, but able to walk unassisted); 4 indicates moderately severe disability (e.g., unable to attend to bodily needs or walk without assistance); 5 indicates severe disability (e.g., requiring constant nursing care, bedridden, incontinent) and 6 indicates death.

In some disclosed embodiments, treating ICH subjects with an effective dose of a FXa variant of the disclosure (e.g., FXa^{I16L}) is effective to reduce neurological impairment caused by ICH as measured using a neurological impairment assessment tool. According to some disclosed embodiments, neurological function assessment tools include the Glasgow Coma Score (GCS), the Glasgow Outcome Scale, extended version (GOS-E), the Barthel Index (BI), the NIH Stroke Scale (NIHSS), or the modified Rankin Scale (mRS). Use of others is also possible. If the GCS, GOS, GOS-E, BI are used, then reduced neurological impairment is reflected by increasing scores.

In some disclosed embodiments, treating an ICH subject with an effective dose of a FXa variant of the disclosure (e.g., FXa^{I16L}) is effective to increase a subject's score on the Glasgow Coma Score by 1, 2, 3, 4, 5, 6, 7, 8, 9, 10, 11, or 12. In other disclosed embodiments, treating an ICH subject with an effective dose of a FXa variant of the disclosure (e.g., FXa^{I16L}) is effective to increase a subject's score on the Glasgow Outcome Scale, including its extended version, by 1, 2, 3, 4, 5, 6, or 7. In yet other disclosed embodiments, treating an ICH subject with an effective dose of a FXa variant of the disclosure (e.g., FXa^{I16L}) is effective to increase a subject's score on the Barthel Index by 1, 2, 3, 4, 5, 6, 7, 8, 9, 10, 11, 12, 13, 14, 15, 16, 17, 18, 19, or 20, or if a wider score range is used, by at least 5, 10, 15, 20, 25, 30, 35, 40, 45, 50, 55, 60, 65, 70, 75, 80, 85, 90, 95, or more.

If the NIHSS or mRS are used, then reduced neurological impairment is reflected by decreasing scores. In some disclosed embodiments, treating an ICH subject with an effective dose of a FXa variant of the disclosure (e.g., FXa^{I16L}) is effective to reduce a subject's score on the NIH Stroke Scale by at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, or 42.

The modified Rankin Scale is commonly used to assess the degree of a subject's disability or dependency caused by ICH. In some disclosed embodiments, treating ICH subjects with an effective dose of a FXa variant of the disclosure (e.g., FXa^{I16L}) is effective to reduce the average mRS score from between about 5 and 6 to between about 4 and 5, from between about 4 and 5 to between about 3 and 4, from between about 3 and 4 to between about 2 and 3, from between about 2 and 3 to between about 1 and 2, from between about 1 and 2 to between about 0 and 1. In other disclosed embodiments, compared no treatment, treatment of ICH subjects with an effective dose of FXa variant increases the proportion of subjects that experience reduction of the average mRS score from between about 5 and 6 to between about 3 and 4, or from between about 3 and 4 to between about 1 and 2.

In other disclosed embodiments, compared to no treatment, treatment of ICH subjects with an effective dose of FXa variant reduces the proportion of subjects with a modified Rankin Scale score of 5 or 6 compared to those that score 0, 1, 2, 3, or 4, or reduces the proportion of subjects with a score of 4, 5, or 6 (characterized as poor) compared to those that score 0, 1, 2, or 3 (characterized as good). In other disclosed embodiments, compared to no treatment, treatment of ICH subjects with an effective dose of FXa variant reduces the proportion of subjects with a score of 2, 3, 4, 5, or 6 compared to those that score 0 or 1. And in other disclosed embodiments, compared with no treatment, treatment of ICH subjects with an effective dose of FXa variant reduces the proportion of subjects that progress to an mRS score of 6 from mRS scores of 2, 3, 4, or 5.

According to some disclosed embodiments, treating ICH subjects with an effective dose of a FXa variant of the disclosure (e.g., FXa^{I16L}) is effective to increase the proportion of ICH subjects for whom neurological impairment has been reduced as a result of treatment by at least about 5%, 10%, 15%, 20%, 25%, 30%, 35%, 40%, 45%, 50%, 55%, 60%, 65%, 70%, 75%, 80%, 85%, 90%, or more compared to untreated control ICH subjects.

Assessment of neurological impairment can be performed using the assessment tools at different times after treatment for ICH. For example, neurological impairment can be measured 7 days, 10 days, 14 days, 20 days, 21 days, 28 days, 30 days, 40 days, 50 days, 60 days, 70 days, 80 days or 90 days or more after treatment for ICH with a FXa variant. Measurement at other times after treatment is also possible according to the knowledge of those of ordinary skill.

### FACTOR XA (FXA) VARIANTS FOR USE IN PREVENTING OR REDUCING EDEMA AFTER ICH

Perihemorrhagic edema (PHE), secondary to ICH, may also contribute to patient morbidity. Without wishing to be bound by any particular theory of operation, products of coagulation and hematoma breakdown after the initial injury are thought to initiate a secondary cascade of damage to the perihemorrhagic brain tissue leading to the development of PHE. The edema, which averages about twice the volume of the underlying hematoma, may contribute to increase in intracranial pressure and mass effects, such as herniation. D. Staykov, et al., Stroke 42:2625-2629 (2011). Increase in edema volume is positively correlated with death and disability. H. Arima, et al., Neurology 73:1963-1968 (2009). Edema volume can be measured using neuroimaging techniques, such as MRI or CT scanning, as described elsewhere herein.

The present disclosure also provides a Factor Xa (FXa) variant for use in for preventing or attenuating perihemorrhagic edema (PHE) associated with ICH by administering to subjects, including human patients, that have experienced ICH a therapeutically effective amount of a FXa variant having zymogenic properties. In some disclosed embodiments the FXa variant is FXa^{I16L}. Edema volume after treatment with a therapeutically effective dose of FXa variant can be reduced by about 5%, 10%, 15%, 20%, 25%, 30%, 35%, 40%, 45%, 50%, 55%, 60%, 65%, 70%, 75%, 80%, 85%, 90% or more compared to the edema volume in ICH patients not treated with FXa variant. In some disclosed embodiments, edema volume is measured using neuroimaging or other technique familiar to those of ordinary skill in the art shortly before FXa variant is administered to the ICH subject. Edema volume can then be measured at least once after treatment with FXa variant begins to monitor the change, if any, in edema volume. In certain disclosed embodiments, edema volume can be measured 1 hour, 2 hours, 3 hours, 4 hours, 5 hours, 6 hours, 9 hours, 12 hours, 18 hours, 24 hours, 48 hours, 72 hours, 96 hours, 5 days, 6 days, 7 days, 14 days, 21 days or more after treatment with FXa variant begins to monitor edema volume changes.

According to other disclosed embodiments, incidence or severity of sequelae of PHE, such as increased intracranial pressure or herniation caused by compression of brain tissue, can be reduced by administering to ICH patients a therapeutically effective amount of a FXa variant having zymogenic properties. In some disclosed embodiments the FXa variant is FXa^{I16L}. Incidence or severity of sequelae of PHE in ICH patients (including but not limited to edema or herniation) treated with a therapeutically effective dose of FXa variant can be reduced by about 5%, 10%, 15%, 20%, 25%, 30%, 35%, 40%, 45%, 50%, 55%, 60%, 65%, 70%, 75%, 80%, 85%, 90% or more compared to that of ICH patients not treated with FXa variant.

### FACTOR XA (FXA) VARIANTS FOR USE IN REDUCING OR PREVENTING HEMATOMA EXPANSION

After the initial hemorrhage caused by ICH about 30% of ICH patients continue to bleed resulting in significant hematoma expansion. Brouwers and Greenberg, Cerebrovasc. Dis. 35:195-201 (2013). Unfortunately, hematoma expansion is associated with worse patient outcome. Davis (2006); Dowlatshahi, et al, Neurology 76:1238-1244 (2011). To treat this subpopulation of ICH patients, compositions comprising FXa variants of the disclosure can be administered at a dose effective to reduce or even prevent hematoma expansion.

A variety of techniques for measuring hematoma volume, initially after ICH and then as it changes over time, are familiar to those of ordinary skill. Non-limiting examples include brain imaging technologies such as CT, CTA, MRI or MRA. Specific methods include ABC/2, ABC/2 with adjusted C values, planimetry and 3D volume rendering. Use of computer software to calculate volume based on data collected during brain scans is common.

Hematoma volume can be calculated based on data collected with the first brain scan used to definitively diagnose ICH, thereby establishing the baseline volume. Hematoma volume can additionally be determined at one or more predetermined times thereafter to monitor whether the hematoma volume is static or expanding, and if expanding, then at what rate.

In some disclosed embodiments, hematoma expansion can be quantified as the percent increase in volume at one or more predetermined times after the baseline brain scan. Non-limiting exemplary relative increases in hematoma volume over some time period compared to baseline include about 5%, 10%, 15%, 20%, 25%, 30%, 35%, 40%, 45%, 50%, 55%, 60%, 65%, 70%, 75%, 80%, 85%, 90%, 95%, 100%, 150%, or more. In other disclosed embodiments, hematoma expansion can be quantified as the absolute increase in volume at one or more predetermined times after the baseline brain scan. Non-limiting exemplary absolute increases in hematoma volume over some time period compared to baseline include about 1 milliliter (ml), 1.5 ml, 2 ml, 2.5 ml, 3 ml, 3.5 ml, 4 ml, 4.5 ml, 5 ml, 5.5 ml, 6 ml, 6.5 ml, 7 ml, 7.5 ml, 8 ml, 8.5 ml, 9 ml, 9.5 ml, 10 ml, 10.5 ml, 11 ml, 11.5 ml, 12 ml, 12.5 ml, 13 ml, 13.5 ml, 14 ml, 14.5 ml, 15 ml, 16 ml, 17 ml, 18 ml, 19 ml, 20 ml, 22 ml, 24 ml, 26 ml, 28 ml, 30 ml, 35 ml, 40 ml, 45 ml, 50 ml, or more.

In non-limiting examples, hematoma volume can be measured at about 1 hour, 2 hours, 3 hours, 4 hours, 5 hours, 6 hours, 9 hours, 12 hours, 15 hours, 18 hours, 24 hours, 36 hours, 48 hours, 60 hours, 72 hours or more after the baseline measurement of hematoma volume to determine whether the volume has increased, and if so by what amount. Multiple scans of the same subject can be taken to track hematoma expansion over time with greater resolution.

According to certain non-limiting disclosed embodiments, hematoma expansion may be defined as occurring when the calculated increase in hematoma volume, if present, exceeds some threshold, either on an absolute volume basis or relative percentage increase in volume. Thus, for example, hematoma expansion may be said to occur when the volume compared to baseline has increased by 3 milliliters (ml) or more, by 6 ml or more, or by 12.5 ml or more, or by some other increase in absolute volume after a predetermined time period. Alternatively, hematoma expansion may be said to occur when the volume compared to baseline has increased by 15% or more, by 20% or more, by 25% or more, by 30% or more, by 33% or more, or by some other percentage increase in volume after a predetermined time period.

According to some disclosed embodiments, treating subjects with ICH with an effective dose of a FXa variant of the disclosure (e.g., FXa^{I16L}) is effective to reduce or prevent hematoma expansion that would otherwise occur, for example, in untreated controls. In some disclosed embodiments, an ICH subject was or is receiving anticoagulant therapy before presenting with ICH. In some other disclosed embodiments, treating a subject with ICH with a FXa variant of the disclosure is effective to reduce the average expansion in hematoma volume that would otherwise have occurred over some time period by at least 1 ml, 1.5 ml, 2 ml, 2.5 ml, 3 ml, 3.5 ml, 4 ml, 4.5 ml, 5 ml, 5.5 ml, 6 ml, 6.5 ml, 7 ml, 7.5 ml, 8 ml, 8.5 ml, 9 ml, 9.5 ml, 10 ml, 10.5 ml, 11 ml, 11.5 ml, 12 ml, 12.5 ml, 13 ml, 13.5 ml, 14 ml, 14.5 ml, 15 ml, 16 ml, 17 ml, 18 ml, 19 ml, 20 ml, 22 ml, 24 ml, 26 ml, 28 ml, 30 ml, 35 ml, 40 ml, 45 ml, 50 ml, or more compared to untreated controls. In other disclosed embodiments, treating subjects with ICH with a FXa variant of the disclosure is effective to reduce the average expansion in hematoma volume that would otherwise have occurred over some time period by at least 5%, 10%, 15%, 20%, 25%, 30%, 35%, 40%, 45%, 50%, 55%, 60%, 65%, 70%, 75%, 80%, 85%, 90%, or 95% compared to untreated controls. In yet other disclosed embodiments, treating subjects with ICH with a FXa variant of the disclosure is effective to reduce the average expansion in hematoma volume that would otherwise have occurred over some time period by at about 5% to 15%, about 10% to 20%, about 15% to 25%, about 20% to 30%, about 25% to 35%, about 30% to 40%, about 35% to 45%, about 40% to 50%, about 45% to 55%, about 50% to 60%, about 55% to 65%, about 60% to 70%, about 65% to 75%, about 70% to 80%, about 75% to 85%, about 80% to 90%, about 85% to 95%, or about 90% to 100%, compared to untreated controls.

According to some disclosed embodiments, treating subjects with ICH with a FXa variant of the disclosure is effective to reduce the proportion of such subjects that experience hematoma expansion. In some disclosed embodiments, the proportion is reduced from about 30% or more to about 25% or less, 20% or less, 15% or less, 10% or less, or 5% or less. In other disclosed embodiments, treatment with a FXa variant of the disclosure reduces the proportion of ICH subjects that experience hematoma expansion over some time period by about 5%, 10%, 15%, 20%, 25%, 30%, 35%, 40%, 45%, 50%, 55%, 60%, 65%, 70%, 75%, 80%, 85%, 90%, 95% or more compared to untreated controls.

In other disclosed embodiments, treating subjects with ICH with a FXa variant of the disclosure is effective to reduce the proportion subjects in whom hematoma volume has increased by 3 ml or more, by 6 ml or more, or by 12.5 ml or more (or by some other amount or more) over some time period by about 5%, 10%, 15%, 20%, 25%, 30%, 35%, 40%, 45%, 50%, 55%, 60%, 65%, 70%, 75%, 80%, 85%, 90%, 95%, or more, compared to untreated controls. In yet other disclosed embodiments, treating subjects with ICH with a FXa variant of the disclosure is effective to reduce the proportion of subjects in whom hematoma volume has increased by 15% or more, by 20% or more, by 25% or more, by 30% or more, by 33% or more (or by some other percentage or more) over some time period by about 5%, 10%, 15%, 20%, 25%, 30%, 35%, 40%, 45%, 50%, 55%, 60%, 65%, 70%, 75%, 80%, 85%, 90%, 95%, or more, compared to untreated controls.

Existence of a so-called "spot sign," identified using CT angiography or other method, is associated with and predictive of hematoma expansion and worse patient outcome. The predictive value of the spot sign for hematoma expansion exists even for patients that present to hospital more than 6 hours after onset of symptoms. Brouwers, et al., Neurocrit Care 17:421-428 (2012).

Spot signs are one or more small foci of contrast enhancement within an acute primary parenchymal hematoma. Without wishing to be bound by any particular theory of operation, the existence of spot signs are believed to be due to active extravasation. Wada, et al, Stroke 38:1257-1262 (2007); Brouwers, et al, Stroke 43:3427-3432 (2012); Demchuk, et al, Lancet Neurol. 11:307-314 (2012).

A scoring system based on spot sign characteristics has been proposed based on the number spot signs, maximum axial dimension, and maximum attenuation. Delgado, et al, Stroke 40:2994-3000 (2009). Where spot signs are present scores range from 1, which corresponds to the presence of one or two spot signs, each of which is less than 5 mm in size and exhibits attenuation of less than 180 HU, up to 4, which corresponds to the presence of three or more spots signs, the largest of which is 5 mm or greater in axial dimension and exhibits maximum attenuation of 180 HU or more. A score of zero indicates that no spot signs were present. The score is highly correlated with risk of hematoma expansion.

According to some disclosed embodiments, subjects with ICH with particular spot sign scores can be treated with a FXa variant of the disclosure. Thus, for example, a subject with ICH and a spot sign score of 4, 3, 2, or 1 can be treated with a FXa variant of the disclosure. In other disclosed embodiments, treating ICH subjects with a FXa variant reduces spot sign scores in particular subjects. Thus, for example, an ICH subject presenting initially with a spot sign score of 4 can have his or her score reduced to 3, 2, 1 or 0 as a result of treatment with a FXa variant. An ICH subject presenting initially with a spot sign score of 3 can have his or her score reduced to 2, 1 or 0 as a result of treatment with a FXa variant. An ICH subject presenting initially with a spot sign score of 2 can have his or her score reduced to 1 or 0 as a result of treatment with a FXa variant. And an ICH subject presenting initially with a spot sign score of 1 can have his or her score reduced to 0 as a result of treatment with a FXa variant.

In certain disclosed embodiments, treating subjects with ICH with a FXa variant of the disclosure is effective to reduce the proportion of such subjects that present with a spot sign score of 4 by about 5%, 10%, 15%, 20%, 25%, 30%, 35%, 40%, 45%, 50%, 55%, 60%, 65%, 70%, 75%, 80%, 85%, 90%, 95% or more compared to untreated controls. In other disclosed embodiments, treating subjects with ICH with a FXa variant of the disclosure is effective to reduce the proportion of such subjects that present with a spot sign score of 3 by about 5%, 10%, 15%, 20%, 25%, 30%, 35%, 40%, 45%, 50%, 55%, 60%, 65%, 70%, 75%, 80%, 85%, 90%, 95% or more compared to untreated controls. According to other disclosed embodiments, treating subjects with ICH with a FXa variant of the disclosure is effective to reduce the proportion of such subjects that present with a spot sign score of 2 by about 5%, 10%, 15%, 20%, 25%, 30%, 35%, 40%, 45%, 50%, 55%, 60%, 65%, 70%, 75%, 80%, 85%, 90%, 95% or more compared to untreated controls. In other disclosed embodiments, treating subjects with ICH with a FXa variant of the disclosure is effective to reduce the proportion of such subjects that present with a spot sign score of 1 by about 5%, 10%, 15%, 20%, 25%, 30%, 35%, 40%, 45%, 50%, 55%, 60%, 65%, 70%, 75%, 80%, 85%, 90%, 95% or more compared to untreated controls. And in yet other disclosed embodiments, treating subjects with ICH with a FXa variant of the disclosure is effective to increase the proportion of such subjects that present with a spot sign score of 0 by about 10%, 20%, 30%, 40%, 50%, 60%, 70%, 80%, 90%, 100%, 150%, 200%, 250%, 300%, 350%, 400% or more compared to untreated controls. According to other disclosed embodiments, treating subjects with ICH with a FXa variant of the disclosure is effective to reduce the incidence of any spot signs by about 5% to 15%, about 10% to 20%, about 15% to 25%, about 20% to 30%, about 25% to 35%, about 30% to 40%, about 35% to 45%, about 40% to 50%, about 45% to 55%, about 50% to 60%, about 55% to 65%, about 60% to 70%, about 65% to 75%, about 70% to 80%, about 75% to 85%, about 80% to 90%, about 85% to 95%, or about 90% to 100%.

According to certain disclosed embodiments, a spot sign score can be determined after neuroimaging at a predetermined time before or after administering a FXa variant of the disclosure. In some disclosed embodiments, for example, spot sign score is determined at 30 min, 45 min, 60 min, 75 min, 90 min, 2 hrs, 2.5 hrs, 3 hrs, 3.5 hrs, 4 hrs, 5 hrs, 6 hrs, 7 hrs, 8 hrs, 9 hrs, 10 hrs, 11 hrs, 12 hrs, 18 hrs, 24 hrs, 30 hrs, 48 hrs, 3 days, 4 days, 5 days, 6 days, 7 days, 8 days, 9 days, 10 days, 11 days, 12 days, 13 days, 14 days, 15 days, 18 days, 3 weeks, 4 weeks, or some other period of time after the first or subsequent administration of FXa variant.

### FACTOR XA (FXA) VARIANTS FOR USE IN TREATING PATIENTS WITH RISK FACTORS FOR ICH

A variety of human patient risk factors have been identified that contribute toward increased risk of ICH. ML Flaherty et al, "The epidemiology of intracerebral hemorrhage," in Intracerebral Hemorrhage, ed. JR Carhuapoma, et al (Cambridge: Cambridge University Press, 2010); Brouwers, et al., Neurocrit Care 17:421-428 (2012); Appelboom, et al., J. Stroke Cereb Dis., 22:713-717 (2013). According to certain disclosed embodiments FXa variants of the disclosure, for example FXa^{I16L}, can be administered in a therapeutically effective amount to human ICH patients having one or more ICH risk factors so as to treat or prevent ICH in such patients.

Age is the most significant risk factor with rates of IHC increasing dramatically among individuals older than 60 years of age. Thus, in some medical uses of the disclosure, a FXa variant, for example FXa^{I16L}, is administered to a patient 60 years of age or older to treat or prevent ICH.

By contrast, hypertension is the most important modifiable risk factor for ICH, present in over 70% of ICH patients. Untreated hypertension is a greater risk factor than hypertension controlled by treatment, and hypertensive patients under treatment who discontinue their medications have greater risk than those who remain on them. Hypertension is a risk factor for hemorrhage in deep hemispheric and brainstem regions, as well as for lobar ICH. The relative impact of hypertension as an ICH risk factor is greater in the young compared to elderly patients. Thus, in some medical uses of the disclosure, a FXa variant, for example FXa^{I16L}, is administered to a hypertensive patient to treat or prevent ICH.

Use of illicit sympathomimetic drugs, for example cocaine and amphetamines, has been associated with ICH. Thus, in some medical uses of the disclosure, a FXa variant, for example FXa^{I16L}, is administered to an ICH patient who used illicit sympathomimetic drugs.

Cerebral amyloid angiopathy (CAA) is also an important risk factor for lobar ICH in the elderly. CAA is characterized by deposition of amyloid protein in the media and adventitia of leptomeningeal arteries, arterioles, capillaries, and sometimes veins. Thus, in some medical uses of the disclosure, a FXa variant, for example FXa^{I16L}, is administered to a patient with cerebral amyloid angiopathy to treat or prevent ICH.

Apolipoprotein E (APOE) ε2 and ε4 alleles are independent risk factors for ICH in the lobar brain regions, potentially as a result of the involvement of these genes in CAA. Brouwers et al, Stroke 43:2120-2125 (2012). Thus, in some medical uses of the disclosure, a FXa variant, for example FXa I16L, is administered to a patient having APOE alleles ε2 or ε4 to treat or prevent ICH.

Vascular malformations, including arteriovenous malformations (AVMs), cavernous malformations, dural arteriovenous fistulae, venous malformations, and capillary telangiectasias, are all associated with ICH. Berry aneurysms can also cause ICH if they bleed into the brain parenchyma as opposed into the subarachnoid space, as is more typical. Thus, in some medical uses of the disclosure, a FXa variant, for example FXa 116L, is administered to a patient with a vascular malformation to treat or prevent ICH.

Use of anticoagulants, such as warfarin, to prevent ischemic stroke, is associated with increased incidence of ICH. The relative risk of ICH in anticoagulated patients is about 7-10 fold greater compared to the general population. Relatedly, use of thrombolytic agents and anticoagulants to effect thrombolysis in myocardial infarction or ischemic stroke is associated with a small but increased risk of ICH. In thrombolysis for myocardial infarction, the majority of hemorrhages are in the lobar regions of the brain. Use of antiplatelet drugs, such as aspirin or clopidogrel, is also a minor risk factor for ICH. Thus, in some medical uses of the disclosure, a FXa variant, for example FXa^{I16L}, is administered to a patient who has been taking anticoagulant (e.g., warfarin) or antiplatelet (e.g., clopidogrel) drugs to treat or prevent ICH.

Microbleeds in the brain parenchyma, detected with gradient echo MRI, are common in patients suffering ICH and are considered markers of small vessel disease and ICH-prone status. Additionally, prior cerebral infarction is associated with a 5-fold to 22-fold increased risk of ICH. Thus, in some medical uses of the disclosure, a FXa variant, for example FXa^{I16L}, is administered to a patient with cerebral microbleeds or who had a prior cerebral infarction to treat or prevent ICH.

Hypocholesterolemia (in contrast with hypercholesterolemia, which is a risk factor for ischemic stroke) is a risk factor for ICH. Thus, in some medical uses of the disclosure, a FXa variant, for example FXa^{I16L}, is administered to a patient with hypocholesterolemia to treat or prevent ICH.

Additional risk factors for ICH include heavy alcohol use, which has been associated with hematoma expansion, and tobacco use. Diabetes is also believed to be a minor risk factor for ICH. Thus, in some medical uses of the disclosure, a FXa variant, for example FXa^{I16L}, is administered to a patient who abuses alcohol or uses tobacco, or to a diabetic patient to treat or prevent ICH. FXa^{I16L}, or another FXa variant, can also be administered to patients in whom trauma, infections, or tumors are sole or contributory causes of ICH to treat or prevent ICH.

### OTHER USES FOR TREATMENT RELATING TO ICH

In other disclosed embodiments, treating subjects with ICH with a FXa variant of the disclosure is effective to reduce disability caused by ICH in a subject. Non-limiting examples of disability include inability or reduced or impaired ability to drive a car, care for oneself without assistance, work, read, speak, move, walk, interact with others, or other types of disability.

According to other disclosed embodiments, treating subjects with ICH with a FXa variant of the disclosure is effective to improve the clinical status of such subjects. Non-limiting examples of clinical status improvement include reduction in the number of days of hospitalization before release to a rehabilitation facility (for example by 1, 2, 3, 4, 5, 6, 7, 8 or more days reduction in hospital stay); downgrading the severity of a patient's status (for example from critical to serious or better, serious to fair or better, fair to good); reduction in the number of days that a ICH patient is admitted to a neuro-intensive care unit (for example by 1, 2, 3, 4, 5, 6, 7, 8 or more days); or reduction in the number of days that a ICH patient is required to be assisted by a mechanical ventilator before regaining the ability to breath unassisted (for example by 1, 2, 3, 4, 5, 6, 7, 8 or more days).

### ADMINISTRATION OF FXA VARIANTS FOR TREATMENT OR PREVENTION OF ICH

FXa variants, such as FXa^{I16L}, should be administered to a patient as soon as possible after onset of ICH symptoms or after diagnosis of ICH has been confirmed. As noted herein, ICH may be suspected upon presentation of certain symptoms and can be confirmed by neuroimaging, e.g., by MRI or CT scanning. According to certain embodiments of the disclosure, FXa variant is administered to a patient within 12, 11, 10, 9, 8, 7, 6, 5, 4, 3, 2, or 1 hours or less after onset of symptoms ICH. In other disclosed embodiments, FXa variant is administered to a patient within 8, 7, 6, 5, 4, 3, 2, 1 hours or less after confirmed diagnosis of ICH by neuroimaging or other technique familiar to those of ordinary skill in the art.

Achieving a target plasma concentration of FXa variant sufficient to treat or prevent ICH is within the knowledge of those ordinarily skilled in the art. In a non-limiting example, estimates of relevant pharmacokinetic parameters, such as subject plasma volume or other parameters, can be made based on upon subject sex, height and weight, or other factors, and used to calculate how much FXa variant needs be administered to achieve a target concentration. After administering FXa variant, plasma concentrations can be monitored according to the knowledge of those ordinarily skilled in the art and this information used to maintain the concentration in any desired range.

Dosage regimens can be adjusted to provide the optimum desired response (e.g., a therapeutic or prophylactic response). For example, a single bolus can be administered, several divided doses can be administered over time or the dose can be proportionally reduced or increased as indicated by the exigencies of the therapeutic situation.

Dosage values may vary depending on such factors as the particular FXa variant used and whether it is used alone or in combination with another therapeutic agent, the unique characteristics of populations, subpopulations, or individual subjects to be treated, and the particular therapeutic or prophylactic effect intended. Other factors that may influence dosage values will be appreciated by those of ordinary skill in the art. Furthermore, for any particular subject, specific dosage regimens may need to be adjusted over time according to the individual need and the professional judgment of the care provider administering or supervising the administration of FXa variant compositions. Thus, the dosage ranges set forth herein are exemplary only and are not intended to limit the scope or practice of the claimed FXa compositions or their medical use.

In certain embodiments, a therapeutically or prophylactically-effective amount of a FXa variant of the disclosure is about 0.0001 to 50 mg/kg, about 0.001 to 50 mg/kg, about 0.001 to 5 mg/kg, about 0.001 to 0.5 mg/kg, about 0.001 to 0.05 mg/kg, about 0.01 to 5 mg/kg or about 0.01 to 0.5 mg/kg. In other disclosed embodiments, a therapeutically or prophylactically-effective blood or plasma concentration of a FXa variant of the disclosure is about 0.0003 to 300 nM, about 0.003 to 300 nM, about 0.03 to 300 nM, about 0.003 to 30 nM, about 0.03 to 30 nM or about 0.3 to 3 nM. Other doses, or blood or plasma concentrations are also possible. The concentration of the FXa variant, for example in blood or plasma, may be measured by any method known in the art.

A FXa variant may be administered, for example in a composition comprising such variant, once or multiple times to a subject until an adequate therapeutic or prophylactic effect is achieved. Where multiple administrations are used they may administered hourly, daily, or at any other appropriate interval, including for example multiple daily doses. Multiple doses may be administered on a schedule such as every 10 minutes, every 15 minutes, every 20 minutes, every 30 minutes, every hour, every 2 hours, every 3 hours, every 4 hours, every 5 hours, every 6 hours, 3 times daily, twice daily, once daily, once every 2 days, once every 3 days, once weekly, or on some other schedule. The FXa variant may also be administered continuously, *e.g*. via a minipump. The FXa variant may be administered, for example, via a parenteral route (*e.g*., intravenously, subcutaneously, intraperitoneally, or intramuscularly). The FXa variant will generally be administered as part of a pharmaceutical composition as described herein.

Administration of FXa variant can be combined with subject monitoring to customize a dosage regime that optimally treats the subject's ICH and effects, such as edema or hematoma expansion. Thus, according to some disclosed embodiments, a dose of FXa variant is administered to an ICH subject who thereafter undergoes neuroimaging to determine if intracerebral bleeding, edema or hematoma expansion has stopped or at least diminished. Where scan results indicate bleeding, edema or hematoma expansion has not stopped or at least improved sufficiently, then another dose of FXa variant can be administered. The process of iteratively dosing and monitoring the subject to assess the effect of FXa variant on intracerebral bleeding, edema or hematoma expansion can be continued until the subject's condition has improved sufficiently. Suitable intervals between dosing and monitoring can be determined according to the knowledge of those ordinarily skilled in the art. Non-limiting examples include 15 minutes, 30 minutes, 45 minutes, 1 hour, 1.5 hours, 2 hours 2.5 hours, 3 hours, 3.5 hours, 4 hours, 5 hours, 6 hours or more.

In another disclosed embodiment, a FXa variant of the disclosure may be co-administered with at least a second active therapeutic agent (combination therapy). In some disclosed embodiments, the second agent can be a different FXa variant of the disclosure. In other disclosed embodiments, the second agent can be another clotting factor or variant thereof, including for example, Factor IX, Factor XIa, Factor XIIa, Factor VIII, Factor VIIa, a procoagulant composition such as FEIBA or prothrombin complex concentrate (PCC), or an anti-fibrinolytic agent such as ε-aminocaproic, tranexemic acid, or aprotinin.

Where a FXa variant of the disclosure is co-administered with at least a second therapeutic agent, they may be administered together at the same time in the same composition or in separate compositions simultaneously or sequentially. Alternatively, FXa variant and the at least second therapeutic agent can be administered at different times in separate compositions. The composition comprising FXa variant can be administered before or after the composition comprising the at least second therapeutic agent. The intervals between sequential administrations can vary with administration of FXa variant and a second therapeutic agent occurring on the same day in certain disclosed embodiments. For instance, FXa variant may be administered once every other day, while the second therapeutic agent is administered once daily, either at the same as the FXa variant, or at a different time on days when both agents are administered.

Combination therapy may be administered multiple times hourly, daily or weekly. Administrations may be administered on a schedule such as every 10 minutes, every 15 minutes, every 20 minutes, every 30 minutes, every hour, every 2 hours, every 3 hours, every 4 hours, every 5 hours, every 6 hours, 3 times daily, twice daily, once daily, once every 2 days, once every 3 days, once weekly, or on some other schedule. The FXa variant may also be administered continuously, e.g. via a minipump. The combination therapy may be administered, for example, via a parenteral route (e.g., intravenously, subcutaneously, intraperitoneally, or intramuscularly).

In other disclosed embodiments, a FXa variant of the disclosure can be administered in concert with another therapy thought to be effective to treat ICH. In non-limiting examples, a FXa variant can be administered before, with or after drugs are administered to reduce the blood pressure or intracranial pressure of a subject with ICH, or surgery, such as to remove a hematoma caused by ICH. In other examples, a FXa variant can be administered before, with or after drugs to prevent or treat seizures or fever, each of which can accompany ICH. R. Sahni and J. Weinberger, Vasc Health Risk Manag 3:701-709 (2007).

In a further aspect, the disclosure provides compositions comprising a FXa variant for use in treating or preventing ICH in a subject. Compositions can comprise a pharmaceutically acceptable carrier, vehicle or other ingredients that are physiologically compatible. Non-limiting examples of such carriers, vehicles and other ingredients include solvents (e.g., water, ethanol, saline, phosphate buffered saline), detergents, surfactants, dispersion media, coatings, antibacterial or antifungal agents, isotonifying agents, absorption delaying agents, sugars (e.g., sucrose, dextrose, lactose), polyalcohols (e.g., glycerol, mannitol, sorbitol), salts (e.g., sodium chloride, potassium chloride), wetting agents, emulsifying agents, preservatives, buffers, and agents capable of enhancing the stability or effectiveness of the FXa variant.

A composition for use according to the disclosure may be in any suitable form for administration to a subject, such as liquid solutions (*e.g.*, injectable and infusible solutions). Compositions can be provided in a pre-mixed format ready for administration to a subject, for example, in a vial or pre-filled syringe. Such formats do not require reconstitution with diluent before administration. Alternatively, compositions can be provided in lyophilized form requiring reconstitution with diluent (e.g., sterile water or saline) before administration. If the latter, diluent can be provided with the lyophilisate in a separate container. According to the knowledge of those of ordinary skill in the art, compositions can be formulated for storage under refrigeration or at room temperature. The form of the composition depends, at least in part, on the intended mode of administration. In certain disclosed embodiments, the mode of administration is parenteral, including for example intravenous, subcutaneous, intraperitoneal, or intramuscular administration.

Parenteral compositions, as well as other types, may be formulated in dosage unit form for ease of administration and uniformity of dosage. Dosage unit form refers to physically discrete units suited as unitary dosages for the subjects to be treated, each unit containing a predetermined quantity of active compound calculated to produce the desired therapeutic effect in association with the required pharmaceutical carrier.

Therapeutic compositions typically must be sterile and stable under the conditions of manufacture and storage. The composition can be formulated as a solution, microemulsion, dispersion, in liposomes, or other ordered structure suitable to high drug concentration. Sterile injectable solutions can be prepared by incorporating the FXa variant in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The disclosure also provides kits comprising a FXa variant or a composition comprising such FXa variant. A kit may further include a diagnostic or one or more additional therapeutic agents. A kit can also include instructions for use in a therapeutic use, as well as packaging materials. A kit can additionally include one or more unit dose forms in containers designed for convenient administration by a care provider. Non-limiting examples include vials or prefilled syringes. Diluents for lyophilized compositions can also be included.

### EXAMPLES

### Example 1

### FXa^{I16L} promotes clot formation in normal plasma

Activity of FXa^{I16L} and plasma derived FXa (pdFXa) were evaluated by measuring activated partial thromboplastin time (aPTT) in citrated pooled plasma isolated from healthy human subjects. The aPTT test was performed by adding a surface activator and dilute phospholipids to citrated plasma. Following an incubation to allow the activation of contact factors (Factor XII, Factor XI, Prekallikrein and high molecular weight kininogen), calcium was added and the clotting time measured. Low levels of FVa and FVIIIa are generated during the assay.

Addition of increasing concentrations of pdFXa or FXa^{I16L} resulted in dose-dependent shortening of the clotting time. The estimated EC₅₀ calculated for FXa^{I16L} and pdFXa were 45 ng/mL and 9 ng/mL, respectively.

Activity of FXa^{I16L} was further examined in citrated plasma isolated from normal mice, rats, and cynomolgus monkeys. Clotting time was measured using the aPTT assay. Similar to what was observed in human plasma, FXa^{I16L} addition resulted in a dose-dependent shortening of the clotting time in all three species. The estimated EC₅₀ calculated for FXa^{I16L} in cynomolgus monkey, mouse and rat plasma was 49, 89, and 91 ng/mL, respectively.

The thrombin generation assay (TGA) measures the initiation phase, activation phase and inactivation phase of thrombin generation. The TGA measures thrombin production in plasma over time following the initiation of coagulation and was performed as previously described (Bunce et al., Blood 117:290-298 (2011)). Under conditions used for these studies, 1 pM tissue factor (TF) was used to initiate coagulation. Thrombin generation (TG) was measured in platelet poor citrated human plasma isolated from healthy subjects. Over the 60-minute time course of the experiment, the addition of FXa^{I16L} or pdFXa resulted in a dose dependent increase in thrombin generation (FIG. 1A, FIG. 1B). Compared to vehicle-treated human plasma, a shortening of the lag time (initiation phase) was observed both for pdFXa and FXa^{I16L}. However, in absolute numbers, the lag time for FXa^{I16L} was longer compared to pdFXa (FIG. 1C). The estimated EC₅₀ calculated for FXa^{I16L} for the described effect on TGA lag time was 9 ng/mL. Peak thrombin generation for FXa^{I16L} and pdFXa were similar, with peak thrombin concentrations ranging from 233-352 nM and 198-349 nM, respectively.

The data summarized above demonstrates that FXa^{I16L} is effective in accelerating clot formation in plasma from non-hemophilic animals, including humans.

### Example 2

### FXa^{I16L} causes hemostasis in a non-hemophilic rodent bleeding models

The ability of FXa^{I16L} to cause hemostasis in non-hemophilic animals was tested by measuring the effect on blood loss after tail cut injury in non-hemophilic mice and rats. Tail clip causes severe bleeding that challenges the coagulation system.

FXa^{I16L} was administered intravenously to normal C57BI/6 mice at different doses (0, 1, 10, 25, 50, 100 and 200 µg/kg). Two minutes later, tails were transected 3 mm from the end and total blood loss until hemostasis was measured in microliters (µl). Control mice were injected with vehicle. Results are shown in FIG. 2A in which data is presented as mean ± SEM, "*" indicates statistical significance at a p value < 0.05, and "***" indicates statistical significance at a p value < 0.001. For vehicle, 5 animals were tested. For animals administered different doses of FXa^{I16L}, 6 were tested at 1 µg/kg, 8 were tested at 10 µg/kg, 8 were tested at 25 µg/kg, 6 were tested at 50 µg/kg, 8 were tested at 100 µg/kg, and 5 were tested at 200 µg/kg.

FXa^{I16L} treatment before tail transection caused a dose-dependent decrease in total blood loss of 12% (1 µg/kg), 16.6% (10 µg/kg), 26.7% (25 µg/kg), 45.3% (50 µg/kg), 62.9% (100 µg/kg), and 69.6% (200 µg/kg) compared to vehicle-treated mice. The estimated ED₅₀ was 46 µg/kg.

In a second study, two minutes after normal male CD-1 mice were treated with 25 µg/kg FXa^{I16L} or vehicle, plasma clotting activity was measured using the activated partial thromboplastin time (aPTT) assay and thrombin generation assay (TGA). In plasma from treated animals, aPTT showed a 67% shorter time to clotting and the TGA lag time was shortened by 85%. Clotting activity in whole blood from CD-1 mice treated with 10 µg/kg or 25 µg/kg FXa^{I16L} or vehicle was also measured with TEG. Two minutes following dosing, TEG R-values of blood from animals treated with both doses of FXa^{I16L} decreased by 70% compared to mice receiving only vehicle.

Hemostatic activity of FXa^{I16L} was also tested in normal male Sprague-Dawley rats in the tail clip severe injury model. Rats pre-treated with FXa^{I16L} showed a dose dependent decrease in bleeding of 5.7% (10 µg/kg), 11.3 % (30 µg/kg), 49.0 % (50 µg/kg), 63.2 % (100 µg/kg) and 59.45 % (200 µg/kg) compared to vehicle treated rats. Results are shown in FIG. 2B in which "*" indicates statistical significance at p value < 0.05. A total of 5-7 rats were used for each dose. Methods were similar as for the experiments using mice. The estimated ED₅₀ was 38 µg/kg. Ex vivo activity in whole blood was measured using TEG. Two minutes after treating rats with 1, 5, 10 or 30 µg/kg FXa^{I16L}, a decrease in TEG-R value of 38.2% and 43.6 % was observed at doses 10 µg/kg and 30 µg/kg, respectively, compared to rats treated with vehicle only.

### Example 3

### FXa^{I16L} reduces hematoma volume in rodent models of ICH

FXa^{I16L} was studied in a mouse model of ICH. In this model, bacterial collagenase is injected into the striatum of the brain stereotactically. F. Schlunk, et al., Stroke 43:246-249 (2012); C. Foerch, et al., Stroke 39:3397-3404 (2008). Proteolytic digestion of the extracellular matrix surrounding capillaries near the injection site results in hemorrhage that mimics hematoma expansion in ICH patients caused by continuous bleeding.

In the experiment using FXa variant, ICH was induced by stereotactic injection of bacterial collagenase into the right striatum of mice. Forty-five minutes after collagenase injection, FXa^{I16L} or vehicle was administered intravenously at different doses. The volume of blood that had leaked into the brain parenchyma as a result of collagenase disruption of capillaries was measured 24 hours post-injury. At that time, blood was flushed from the vasculature, brains were removed and homogenized, and the hemoglobin content of the homogenized brain tissue was quantitated by comparison to a standard curve.

Treatment with FXa^{I16L} reduced the hematoma volume in mice in a dose responsive manner compared to mice treated with vehicle only. Results are shown in FIG. 3A in which "*" indicates statistical significance at a p value < 0.05. Estimated ED₅₀ = 3.05 µg/kg. Animals tested per group ranged between 7 and 15. FIG. 3B shows the percent decrease in hematoma volume compared to vehicle in relation to dose.

Using similar methods as those used in mice, ICH was induced in male Sprague Dawley rats. Fifteen minutes after collagenase injection, rats were intravenously administered different doses of FXa^{I16L}, the procoagulant composition Factor eight inhibitor bypass activity (FEIBA) at 100 U/kg, or vehicle alone. Hematoma volume based on brain hemoglobin content was determined at 2 hours post-injury.

Treatment with FXa^{I16L} reduced the hematoma volume in rats compared to rats treated with vehicle only. Results are shown in FIG. 4A in which "*" indicates statistical significance at a p value < 0.05. FIG. 4B shows the percent decrease in hematoma volume compared to vehicle in relation to dose. Although not as marked as in mice, a trend towards dose responsiveness was evident.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclature used in connection with, and techniques of, cell and tissue culture, molecular biology, immunology, microbiology, genetics and protein and nucleic acid chemistry and hybridization described herein are those well known and commonly used in the art.

The medical uses and techniques disclosed herein are generally performed according to conventional methods well known in the art and as described in various general and more specific references that are cited and discussed throughout the present specification unless otherwise indicated. See, e.g., Sambrook J. & Russell D.. Molecular Cloning: A Laboratory Manual, 3rd ed., Cold Spring Harbor Laboratory Press, Cold Spring Harbor, N.Y. (2000); Ausubel et al., Short Protocols in Molecular Biology: A Compendium of Methods from Current Protocols in Molecular Biology, Wiley, John & Sons, Inc. (2002); Harlow and Lane Using Antibodies: A Laboratory Manual, Cold Spring Harbor Laboratory Press, Cold Spring Harbor, N.Y. (1998); and Coligan et al., Short Protocols in Protein Science, Wiley, John & Sons, Inc. (2003). Enzymatic reactions and purification techniques are performed according to manufacturer's specifications, as commonly accomplished in the art or as described herein. The nomenclature used in connection with, and the laboratory procedures and techniques of, analytical chemistry, synthetic organic chemistry, and medicinal and pharmaceutical chemistry described herein are those well known and commonly used in the art.

.

Throughout this specification and claims, the word "comprise," or variations such as "comprises" or "comprising," will be understood to imply the inclusion of a stated integer or group of integers but not the exclusion of any other integer or group of integers.

## Claims

1. A Factor Xa (FXa) variant for use in the treatment of intracerebral haemorrhage (ICH), wherein Factor Xa (FXa) variant includes at least one substitution mutation selected from the group consisting of:
a) the amino acid at the position corresponding to 235 in SEQ ID NO:1 is substituted with Thr, Leu, Phe, Asp or Gly; and
b) the amino acid at the position corresponding to 236 in SEQ ID NO:1 is substituted with Leu, Ala, or Gly.

2. The Factor Xa (FXa) variant for use according to claim 1, wherein the
(i) likelihood that a subject with ICH will die is reduced,
(ii) the brain function of a subject with ICH is improved,
(iii) neurological impairment of a subject with ICH is reduced,
(iv) perihemorrhagic edema (PHE) is reduced, or
(v) hematoma volume expansion is reduced or prevented.

3. The Factor Xa (FXa) variant for use according to claim 1 or 2, wherein the amino acid at the position corresponding to 235 in SEQ ID NO:1 is substituted with Leu.

4. The Factor Xa (FXa) variant for use according to claim 2(ii) or 3, wherein improved brain function is associated with;
(a) reducing elevated intracranial pressure (ICP) caused by ICH equal to or below a pressure value selected from the group consisting of 80 mm Hg, 70 mm Hg, 60 mm Hg, 50 mm Hg, 40 mm Hg, 30 mm Hg, 20 mm Hg, and 10 mm Hg,
(b) maintaining cerebral perfusion pressure (CPP) at a pressure value equal to or above a value selected from the group consisting of 40 mm Hg, 50 mm Hg, 60 mm Hg, 70 mm Hg, 80 mm Hg, 90 mm Hg, 100 mm Hg, 110, mm Hg, and 120 mm Hg,
(c) maintaining brain tissue oxygen tension (PbtO2) at a pressure value equal to or above a value selected from the group consisting of 6 mm Hg, 8 mm Hg, 10 mm Hg, 12 mm Hg, 14 mm Hg, 16 mm Hg, 18 mm Hg, 20, mm Hg, 22 mm Hg, and 24 mm Hg,
(d) reducing the ratio of the concentrations of lactate to pyruvate (LAR) equal to or below a value selected from the group consisting of 60, 50, 40, 30, and 20, or
(e) reducing the ratio of the cerebrovascular pressure reactivity index (PRx) equal to or below a value selected from the group consisting of 0.5, 0.4, 0.3, 0.2, 0.1, 0.0, -0.1, -0.2, and -0.3.

5. The Factor Xa (FXa) variant for use according to claim 2(iii) or 3, wherein neurological impairment is assessed using;
(a) the Glasgow Coma Score and wherein as a result of treatment the subject's score improves from 3 to 4 or higher, from 4 to 5 or higher, from 5 to 6 or higher, from 6 to 7 or higher, from 7 to 8 or higher, from 8 to 9 or higher, from 9 to 10 or higher, from 10 to 11 or higher, from 11 to 12 or higher, from 12 to 13 or higher, from 13 to 14 or higher, or from 14 to 15,
(b) the Glasgow Outcome Scale extended version and wherein as a result of treatment the average score of treated subjects improves to 1 or better, 2 or better, 3 or better, 4 or better, 5 or better, 6 or better, 7 or better, or 8,
(c) the Barthel Index and wherein as a result of treatment the average score of treated subjects improves to 10 or better, 20 or better, 30 or better, 40 or better, 50 or better, 60 or better, 70 or better, or 80 or better, or 90 or better,
(d) the NIH Stroke Scale and wherein as a result of treatment the average score of treated subjects is reduced from 42 to 41 or less, 41 to 40 or less, 40 to 39 or less, 39 to 38 or less, 38 to 37 or less, 37 to 36 or less, 36 to 35 or less, 35 to 34 or less, 34 to 33 or less, 33 to 32 or less, 32 to 31 or less, 31 to 30 or less, 30 to 29 or less, 29 to 28 or less, 28 to 27 or less, 27 to 26 or less, 26 to 25 or less, 25 to 24 or less, 24 to 23 or less, 23 to 22 or less, 22 to 21 or less, 21 to 20 or less, 20 to 19 or less, 19 to 18 or less, 18 to 17 or less, 17 to 16 or less, 16 to 15 or less, 15 to 14 or less, 14 to 13 or less, 13 to 12 or less, 12 to 11 or less, 11 to 10 or less, 10 to 9 or less, 9 to 8 or less, 8 to 7 or less, 7 to 6 or less, 6 to 5 or less, 5 to 4 or less, 4 to 3 or less, 3 to 2 or less, 2 to 1 or less, or 1 to 0,
(e) the modified Rankin Scale and wherein as a result of treatment the average score of treated subjects is reduced from between about 5 and 6 to between about 4 and 5, from between about 4 and 5 to between about 3 and 4, from between about 3 and 4 to between about 2 and 3, from between about 2 and 3 to between about 1 and 2, or from between about 1 and 2 to between about 0 and 1,
(f) the modified Rankin Scale and wherein as a result of treatment the proportion of treated subjects with a modified Rankin Scale score of 5 or 6 is reduced compared to those that score 0, 1, 2, 3, or 4, or
(g) the modified Rankin Scale and wherein as a result of treatment the proportion of treated subjects with a modified Rankin Scale score of 4, 5 or 6 is reduced compared to those that score 0, 1, 2, or 3.

6. The Factor Xa (FXa) variant for use according to claim 2(iv) or 3, wherein as a result of treatment the average volume of PHE in subjects with ICH is reduced by at least about 5%, 10%, 15%, 20%, 25%, 30%, 35%, 40%, 45%, 50%, 55%, 60%, 65%, 70%, 75%, 80%, 85%, 90%, or 95% compared to untreated controls, or the average increase in intracranial pressure caused by PHE in subjects with ICH is reduced by at least about 5%, 10%, 15%, 20%, 25%, 30%, 35%, 40%, 45%, 50%, 55%, 60%, 65%, 70%, 75%, 80%, 85%, 90%, or 95% compared to untreated controls.

7. The Factor Xa (FXa) variant for use according to claim 2(v) or 3, wherein,
(a) as a result of treatment the average hematoma volume expansion in subjects with ICH is reduced by at least about 5%, 10%, 15%, 20%, 25%, 30%, 35%, 40%, 45%, 50%, 55%, 60%, 65%, 70%, 75%, 80%, 85%, 90%, or 95% compared to untreated controls,
(b) as a result of treatment the average hematoma volume expansion in subjects with ICH is reduced by at least about 1 ml, 1.5 ml, 2 ml, 2.5 ml, 3 ml, 3.5 ml, 4 ml, 4.5 ml, 5 ml, 5.5 ml, 6 ml, 6.5 ml, 7 ml, 7.5 ml, 8 ml, 8.5 ml, 9 ml, 9.5 ml, 10 ml, 10.5 ml, 11 ml, 11.5 ml, 12 ml, 12.5 ml, 13 ml, 13.5 ml, 14 ml, 14.5 ml, 15 ml, 16 ml, 17 ml, 18 ml, 19 ml, 20 ml, 22 ml, 24 ml, 26 ml, 28 ml, 30 ml, 35 ml, 40 ml, 45 ml, or 50 ml, compared to untreated controls,
(c) the proportion of subjects with ICH in whom hematoma volume has expanded 3 ml or more, 6 ml or more, or 12.5 ml or more is reduced by at least about 5%, 10%, 15%, 20%, 25%, 30%, 35%, 40%, 45%, 50%, 55%, 60%, 65%, 70%, 75%, 80%, 85%, 90%, or 95%, compared to untreated controls,
(d) the proportion of subjects with ICH in whom hematoma volume has expanded by 15% or more, by 20% or more, by 25% or more, by 30% or more, by 33% or more is reduced by at least about 5%, 10%, 15%, 20%, 25%, 30%, 35%, 40%, 45%, 50%, 55%, 60%, 65%, 70%, 75%, 80%, 85%, 90%, or 95%, compared to untreated controls,
(e) the proportion of ICH subjects having a spot sign score of 4 after treatment is reduced by at least about 5%, 10%, 15%, 20%, 25%, 30%, 35%, 40%, 45%, 50%, 55%, 60%, 65%, 70%, 75%, 80%, 85%, 90%, or 95%, compared to untreated controls,
(f) the proportion of ICH subjects having a spot sign score of 3 after treatment is reduced by at least about 5%, 10%, 15%, 20%, 25%, 30%, 35%, 40%, 45%, 50%, 55%, 60%, 65%, 70%, 75%, 80%, 85%, 90%, or 95%, compared to untreated controls,
(g) the proportion of ICH subjects having a spot sign score of 2 after treatment is reduced by at least about 5%, 10%, 15%, 20%, 25%, 30%, 35%, 40%, 45%, 50%, 55%, 60%, 65%, 70%, 75%, 80%, 85%, 90%, or 95%, compared to untreated controls, or
(h) the proportion of ICH subjects having a spot sign score of 1 after treatment is reduced by at least about 5%, 10%, 15%, 20%, 25%, 30%, 35%, 40%, 45%, 50%, 55%, 60%, 65%, 70%, 75%, 80%, 85%, 90%, or 95%, compared to untreated controls.

8. The Factor Xa (FXa) variant for use according to claim 7(e) to (h), wherein the spot sign score is determined a period of time after first administration of FXa variant selected from the group consisting of 30 min, 45 min, 60 min, 75 min, 90 min, 2 hrs, 2.5 hrs, 3 hrs, 3.5 hrs, 4 hrs, 5 hrs, 6 hrs, 7 hrs, 8 hrs, 9 hrs, 10 hrs, 11 hrs, 12 hrs, 18 hrs, 24 hrs, 30 hrs, 48 hrs, 3 days, 4 days, 5 days, 6 days, 7 days, 8 days, 9 days, 10 days, 11 days, 12 days, 13 days, 14 days, 15 days, 18 days, 3 weeks, and 4 weeks.

9. The Factor Xa (FXa) variant for use according to any preceding claim, wherein the intracerebral hemorrhage occurs in a brain region selected from the group consisting of cerebrum, frontal lobe, parietal lobe, occipital lobe, temporal lobe, cerebellum, brain stem, midbrain, pons, medulla, pituitary gland, hypothalamus, thalamus, hippocampus, amygdala, claustrum, and basal ganglia.

## Patentansprüche

1. Faktor-Xa (FXa)-Variante zur Verwendung bei der Behandlung von intrazerebraler Blutung (ICH, intracerebral haemorrhage), wobei die Faktor-Xa (FXa)-Variante wenigstens eine Substitutionsmutation umfasst, die ausgewählt ist aus der Gruppe, bestehend aus:
a) die Aminosäure an der Position, die 235 in SEQ ID NO:1 entspricht, ist durch Thr, Leu, Phe, Asp oder Gly ersetzt und
b) die Aminosäure an der Position, die 236 in SEQ ID NO:1 entspricht, ist durch Leu, Ala oder Gly ersetzt.

2. Faktor-Xa (FXa)-Variante zur Verwendung gemäß Anspruch 1, wobei
(i) die Wahrscheinlichkeit, dass ein Subjekt mit ICH sterben wird, verringert ist,
(ii) die Gehirnfunktion eines Subjekts mit ICH verbessert ist,
(iii) die neurologische Beeinträchtigung eines Subjekts mit ICH verringert ist,
(iv) das perihämorrhagische Ödem (PHE) verringert ist oder
(v) die Hämatomvolumenexpansion verringert ist oder dieser vorgebeugt wird.

3. Faktor-Xa (FXa)-Variante zur Verwendung gemäß Anspruch 1 oder 2, wobei die Aminosäure an der Position, die 235 in SEQ ID NO:1 entspricht, durch Leu ersetzt ist.

4. Faktor-Xa (FXa)-Variante zur Verwendung gemäß Anspruch 2(ii) oder 3, wobei die verbesserte Gehirnfunktion in Verbindung steht mit:
(a) dem Verringern von erhöhtem intrakraniellen Druck (ICP), der durch ICH verursacht ist, auf einen Wert gleich oder unterhalb eines Druckwertes, ausgewählt aus der Gruppe, bestehend aus 80 mm Hg, 70 mm Hg, 60 mm Hg, 50 mm Hg, 40 mm Hg, 30 mm Hg, 20 mm Hg und 10 mm Hg,
(b) Aufrechterhalten von Hirnperfusionsdruck (CPP) bei einem Druckwert gleich oder oberhalb eines Wertes, ausgewählt aus der Gruppe, bestehend aus 40 mm Hg, 50 mm Hg, 60 mm Hg, 70 mm Hg, 80 mm Hg, 90 mm Hg, 100 mm Hg, 110 mm Hg und 120 mm Hg,
(c) Aufrechterhalten von Gehirngewebesauerstoffdruck (PbtO2) bei einem Druckwert gleich oder oberhalb eines Wertes, ausgewählt aus der Gruppe, bestehend aus 6 mm Hg, 8 mm Hg, 10 mm Hg, 12 mm Hg, 14 mm Hg, 16 mm Hg, 18 mm Hg, 20 mm Hg, 22 mm Hg und 24 mm Hg,
(d) Verringern des Verhältnisses der Konzentrationen von Lactat zu Pyruvat (LAR) auf einen Wert gleich oder unterhalb eines Wertes, ausgewählt aus der Gruppe, bestehend aus 60, 50, 40, 30 und 20, oder
(e) Verringern des Verhältnisses des Zerebrovaskulärdruck-Reaktivitätsindex (PRx) auf einen Wert gleich oder unterhalb eines Wertes, ausgewählt aus der Gruppe, bestehend aus 0,5, 0,4, 0,3, 0,2, 0,1, 0,0, -0,1, -0,2 und -0,3.

5. Faktor-Xa (FXa)-Variante zur Verwendung gemäß Anspruch 2(iii) oder 3, wobei die neurologische Beeinträchtigung beurteilt wird unter Verwendung:
(a) der Glasgow-Coma-Skala (Glasgow Coma Score), und wobei sich als ein Ergebnis der Behandlung die Punktzahl des Subjekts von 3 auf 4 oder höher, von 4 auf 5 oder höher, von 5 auf 6 oder höher, von 6 auf 7 oder höher, von 7 auf 8 oder höher, von 8 auf 9 oder höher, von 9 auf 10 oder höher, von 10 auf 11 oder höher, von 11 auf 12 oder höher, von 12 auf 13 oder höher, von 13 auf 14 oder höher oder von 14 auf 15 verbessert,
(b) der erweiterten Version der Glasgow-Outcome-Skala, und wobei sich als ein Ergebnis der Behandlung die durchschnittliche Punktzahl von behandelten Subjekten auf 1 oder besser, 2 oder besser, 3 oder besser, 4 oder besser, 5 oder besser, 6 oder besser, 7 oder besser oder 8 verbessert,
(c) des Barthel-Index, und wobei sich als ein Ergebnis der Behandlung die durchschnittliche Punktzahl von behandelten Subjekten auf 10 oder besser, 20 oder besser, 30 oder besser, 40 oder besser, 50 oder besser, 60 oder besser, 70 oder besser oder 80 oder besser oder 90 oder besser verbessert,
(d) der NIH-Schlaganfall-Skala (NIH Stroke Scale), und wobei als ein Ergebnis der Behandlung die durchschnittliche Punktzahl von behandelten Subjekten von 42 auf 41 oder weniger, von 41 auf 40 oder weniger, von 40 auf 39 oder weniger, von 39 auf 38 oder weniger, von 38 auf 37 oder weniger, von 37 auf 36 oder weniger, von 36 auf 35 oder weniger, von 35 auf 34 oder weniger, von 34 auf 33 oder weniger, von 33 auf 32 oder weniger, von 32 auf 31 oder weniger, von 31 auf 30 oder weniger, von 30 auf 29 oder weniger, von 29 auf 28 oder weniger, von 28 auf 27 oder weniger, von 27 auf 26 oder weniger, von 26 auf 25 oder weniger, von 25 auf 24 oder weniger, von 24 auf 23 oder weniger, von 23 auf 22 oder weniger, von 22 auf 21 oder weniger, von 21 auf 20 oder weniger, von 20 auf 19 oder weniger, von 19 auf 18 oder weniger, von 18 auf 17 oder weniger, von 17 auf 16 oder weniger, von 16 auf 15 oder weniger, von 15 auf 14 oder weniger, von 14 auf 13 oder weniger, von 13 auf 12 oder weniger, von 12 auf 11 oder weniger, von 11 auf 10 oder weniger, von 10 auf 9 oder weniger, von 9 auf 8 oder weniger, von 8 auf 7 oder weniger, von 7 auf 6 oder weniger, von 6 auf 5 oder weniger, von 5 auf 4 oder weniger, von 4 auf 3 oder weniger, von 3 auf 2 oder weniger, von 2 auf 1 oder weniger oder von 1 auf 0 verringert ist,
(e) der modifizierten Rankin-Skala, und wobei als ein Ergebnis der Behandlung die durchschnittliche Punktzahl von behandelten Subjekten von zwischen etwa 5 und 6 auf zwischen etwa 4 und 5, von zwischen etwa 4 und 5 auf zwischen etwa 3 und 4, von zwischen etwa 3 und 4 auf zwischen etwa 2 und 3, von zwischen etwa 2 und 3 auf zwischen etwa 1 und 2 oder von zwischen etwa 1 und 2 auf zwischen etwa 0 und 1 verringert ist,
(f) der modifizierten Rankin-Skala, und wobei als ein Ergebnis der Behandlung der Anteil von behandelten Subjekten mit einer Punktzahl der modifizierten Rankin-Skala von 5 oder 6 im Vergleich zu denjenigen, die eine Punktzahl von 0, 1, 2, 3 oder 4 erreichen, verringert ist, oder
(g) der modifizierten Rankin-Skala, und wobei als ein Ergebnis der Behandlung der Anteil von behandelten Subjekten mit einer Punktzahl der modifizierten Rankin-Skala von 4, 5 oder 6 im Vergleich zu denjenigen, die eine Punktzahl von 0, 1, 2 oder 3 erreichen, verringert ist.

6. Faktor-Xa (FXa)-Variante zur Verwendung gemäß Anspruch 2(iv) oder 3, wobei als ein Ergebnis der Behandlung das durchschnittliche Volumen von PHE bei Subjekten mit ICH im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist, oder die durchschnittliche Zunahme von intrakraniellem Druck, der durch PHE verursacht ist, bei Subjekten mit ICH im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist.

7. Faktor-Xa (FXa)-Variante zur Verwendung gemäß Anspruch 2(v) oder 3, wobei
(a) als ein Ergebnis der Behandlung die durchschnittliche Hämatomvolumenexpansion bei Subjekten mit ICH im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist,
(b) als ein Ergebnis der Behandlung die durchschnittliche Hämatomvolumenexpansion bei Subjekten mit ICH im Vergleich zu unbehandelten Kontrollen um wenigstens etwa 1 ml, 1,5 ml, 2 ml, 2,5 ml, 3 ml, 3,5 ml, 4 ml, 4,5 ml, 5 ml, 5,5 ml, 6 ml, 6,5 ml, 7 ml, 7,5 ml, 8 ml, 8,5 ml, 9 ml, 9,5 ml, 10 ml, 10,5 ml, 11 ml, 11,5 ml, 12 ml, 12,5 ml, 13 ml, 13,5 ml, 14 ml, 14,5 ml, 15 ml, 16 ml, 17 ml, 18 ml, 19 ml, 20 ml, 22 ml, 24 ml, 26 ml, 28 ml, 30 ml, 35 ml, 40 ml, 45 ml oder 50 ml verringert ist,
(c) der Anteil an Subjekten mit ICH, bei denen sich das Hämatomvolumen um 3 ml oder mehr, 6 ml oder mehr oder 12,5 ml oder mehr vergrößert hat, im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist,
(d) der Anteil an Subjekten mit ICH, bei denen sich das Hämatomvolumen um 15% oder mehr, um 20% oder mehr, um 25% oder mehr, um 30% oder mehr, um 33% oder mehr vergrößert hat, im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist,
(e) der Anteil von ICH-Subjekten mit einem Spot-Sign-Score von 4 nach der Behandlung im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist,
(f) der Anteil von ICH-Subjekten mit einem Spot-Sign-Score von 3 nach der Behandlung im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist,
(g) der Anteil von ICH-Subjekten mit einem Spot-Sign-Score von 2 nach der Behandlung im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist, oder
(h) der Anteil von ICH-Subjekten mit einem Spot-Sign-Score von 1 nach der Behandlung im Vergleich zu unbehandelten Kontrollen um wenigstens etwa 5%, 10%, 15%, 20%, 25%, 30%, 35%, 40%, 45%, 50%, 55%, 60%, 65%, 70%, 75%, 80%, 85%, 90% oder 95% verringert ist.

8. Faktor-Xa (FXa)-Variante zur Verwendung gemäß Anspruch 7(e) bis (h), wobei der Spot-Sign-Score zu einem Zeitraum nach der ersten Verabreichung von FXa-Variante, ausgewählt aus der Gruppe, bestehend aus 30 min, 45 min, 60 min, 75 min, 90 min, 2 Std., 2,5 Std., 3 Std., 3,5 Std., 4 Std., 5 Std., 6 Std., 7 Std., 8 Std., 9 Std., 10 Std., 11 Std., 12 Std., 18 Std., 24 Std., 30 Std., 48 Std., 3 Tagen, 4 Tagen, 5 Tagen, 6 Tagen, 7 Tagen, 8 Tagen, 9 Tagen, 10 Tagen, 11 Tagen, 12 Tagen, 13 Tagen, 14 Tagen, 15 Tagen, 18 Tagen, 3 Wochen und 4 Wochen, bestimmt wird.

9. Faktor-Xa (FXa)-Variante zur Verwendung gemäß einem beliebigen vorangehenden Anspruch, wobei die intrazerebrale Blutung in einer Gehirnregion, ausgewählt aus der Gruppe, bestehend aus Cerebrum, Frontallappen, Parietallappen, Occipitallappen, Temporallappen, Cerebellum, Gehirnstamm, Mittelhirn, Gehirnbrücke, Medulla, Hypophyse, Hypothalamus, Thalamus, Hippocampus, Amygdala, Claustrum und Basalganglien, auftritt.

## Revendications

1. Variant de facteur Xa (FXa) à utiliser dans le traitement de l'hémorragie intracérébrale (HIC), dans lequel le variant de facteur Xa (FXa) inclut au moins une mutation de substitution choisie dans le groupe constitué par :
a) l'acide aminé à la position correspondant à 235 dans SEQ ID N° 1 est substitué par Thr, Leu, Phe, Asp ou Gly ; et
b) l'acide aminé à la position correspondant à 236 dans SEQ ID N° 1 est substitué par Leu, Ala, ou Gly.

2. Variant de facteur Xa (FXa) à utiliser selon la revendication 1, dans lequel
(i) la vraisemblance qu'un sujet atteint de HIC mourra est réduite,
(ii) la fonction cérébrale d'un sujet atteint de HIC est améliorée,
(iii) l'altération neurologique d'un sujet atteint de HIC est réduite,
(iv) un œdème périhémorragique (PHE) est réduit, ou
(v) un accroissement de volume d'hématome est réduit ou empêché

3. Variant de facteur Xa (FXa) à utiliser selon la revendication 1 ou 2, dans lequel l'acide aminé à la position correspondant à 235 dans SEQ ID N° 1 est substitué par Leu.

4. Variant de facteur Xa (FXa) à utiliser selon la revendication 2(ii) ou 3, dans lequel la fonction cérébrale améliorée est associée à ;
(a) la réduction d'une pression intracrânienne (PIC) élevée provoquée par l'HIC inférieure ou égale à une valeur de pression choisie dans le groupe constitué par 80 mm Hg, 70 mm Hg, 60 mm Hg, 50 mm Hg, 40 mm Hg, 30 mm Hg, 20 mm Hg, et 10 mm Hg,
(b) le maintien d'une pression de perfusion cérébrale (PPC) à une valeur de pression supérieure ou égale à une valeur choisie dans le groupe constitué par 40 mm Hg, 50 mm Hg, 60 mm Hg, 70 mm Hg, 80 mm Hg, 90 mm Hg, 100 mm Hg, 110 mm Hg, et 120 mm Hg,
(c) le maintien d'une pression tissulaire cérébrale en oxygène (PtiO2) à une valeur de pression supérieure ou égale à une valeur choisie dans le groupe constitué par 6 mm Hg, 8 mm Hg, 10 mm Hg, 12 mm Hg, 14 mm Hg, 16 mm Hg, 18 mm Hg, 20 mm Hg, 22 mm Hg, et 24 mm Hg,
(d) la réduction du rapport des concentrations de lactate sur pyruvate (LAR) inférieur ou égal à une valeur choisie dans le groupe constitué par 60, 50, 40, 30, et 20, ou
(e) la réduction du rapport de l'indice de réactivité de pression cérébrovasculaire (PRx) inférieur ou égal à une valeur choisie dans le groupe constitué par 0,5, 0,4, 0,3, 0,2, 0,1, 0,0, -0,1, -0,2, et -0,3.

5. Variant de facteur Xa (FXa) à utiliser selon la revendication 2(iii) ou 3, dans lequel l'altération neurologique est estimée en utilisant ;
(a) le score de Glasgow et dans lequel grâce au traitement, le score du sujet s'améliore de 3 à 4 ou plus, de 4 à 5 ou plus, de 5 à 6 ou plus, de 6 à 7 ou plus, de 7 à 8 ou plus, de 8 à 9 ou plus, de 9 à 10 ou plus, de 10 à 11 ou plus, de 11 à 12 ou plus, de 12 à 13 ou plus, de 13 à 14 ou plus, ou de 14 à 15,
(b) la version étendue de l'échelle de Glasgow et dans lequel grâce au traitement, le score moyen de sujets traités s'améliore à 1 ou plus, 2 ou plus, 3 ou plus, 4 ou plus, 5 ou plus, 6 ou plus, 7 ou plus, ou 8,
(c) l'indice de Barthel et dans lequel grâce au traitement, le score moyen de sujets traités s'améliore à 10 ou plus, 20 ou plus, 30 ou plus, 40 ou plus, 50 ou plus, 60 ou plus, 70 ou plus, ou 80 ou plus, ou 90 ou plus,
(d) l'échelle NIHSS et dans lequel grâce au traitement, le score moyen de sujets traités est réduit de 42 à 41 ou moins, 41 à 40 ou moins, 40 à 39 ou moins, 39 à 38 ou moins, 38 à 37 ou moins, 37 à 36 ou moins, 36 à 35 ou moins, 35 à 34 ou moins, 34 à 33 ou moins, 33 à 32 ou moins, 32 à 31 ou moins, 31 à 30 ou moins, 30 à 29 ou moins, 29 à 28 ou moins, 28 à 27 ou moins, 27 à 26 ou moins, 26 à 25 ou moins, 25 à 24 ou moins, 24 à 23 ou moins, 23 à 22 ou moins, 22 à 21 ou moins, 21 à 20 ou moins, 20 à 19 ou moins, 19 à 18 ou moins, 18 à 17 ou moins, 17 à 16 ou moins, 16 à 15 ou moins, 15 à 14 ou moins, 14 à 13 ou moins, 13 à 12 ou moins, 12 à 11 ou moins, 11 à 10 ou moins, 10 à 9 ou moins, 9 à 8 ou moins, 8 à 7 ou moins, 7 à 6 ou moins, 6 à 5 ou moins, 5 à 4 ou moins, 4 à 3 ou moins, 3 à 2 ou moins, 2 à 1 ou moins, ou 1 à 0,
(e) l'échelle de Rankin modifiée et dans lequel grâce au traitement, le score moyen de sujets traités est réduit d'entre environ 5 et 6 à entre environ 4 et 5, d'entre environ 4 et 5 à entre environ 3 et 4, d'entre environ 3 et 4 à entre environ 2 et 3, d'entre environ 2 et 3 à entre environ 1 et 2, ou d'entre environ 1 et 2 à entre environ 0 et 1,
(f) l'échelle de Rankin modifiée et dans lequel grâce au traitement, la proportion de sujets traités avec un score d'échelle de Rankin modifiée de 5 ou 6 est réduite en comparaison à ceux dont le score est 0, 1, 2, 3, ou 4, ou
(g) l'échelle de Rankin modifiée et dans lequel grâce au traitement, la proportion de sujets traités avec un score d'échelle de Rankin modifiée de 4, 5 ou 6 est réduite en comparaison à ceux dont le score est 0, 1, 2, ou 3.

6. Variant de facteur Xa (FXa) à utiliser selon la revendication 2(iv) ou 3, dans lequel grâce au traitement, le volume moyen de PHE chez des sujets atteints de HIC est réduit d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 % en comparaison à des témoins non traités, ou l'augmentation moyenne de pression intracrânienne provoquée par PHE chez des sujets atteints de HIC est réduite d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 % en comparaison à des témoins non traités.

7. Variant de facteur Xa (FXa) à utiliser selon la revendication 2(v) ou 3, dans lequel,
(a) grâce au traitement, l'accroissement de volume d'hématome moyen chez des sujets atteints de HIC est réduit d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 % en comparaison à des témoins non traités,
(b) grâce au traitement, l'accroissement de volume d'hématome moyen chez des sujets atteints de HIC est réduit d'au moins environ 1 ml, 1,5 ml, 2 ml, 2,5 ml, 3 ml, 3,5 ml, 4 ml, 4,5 ml, 5 ml, 5,5 ml, 6 ml, 6,5 ml, 7 ml, 7,5 ml, 8 ml, 8,5 ml, 9 ml, 9,5 ml, 10 ml, 10,5 ml, 11 ml, 11,5 ml, 12 ml, 12,5 ml, 13 ml, 13,5 ml, 14 ml, 14,5 ml, 15 ml, 16 ml, 17 ml, 18 ml, 19 ml, 20 ml, 22 ml, 24 ml, 26 ml, 28 ml, 30 ml, 35 ml, 40 ml, 45 ml, ou 50 ml, en comparaison à des témoins non traités,
(c) la proportion de sujets atteints de HIC chez lesquels le volume d'hématome a crû de 3 ml ou plus, 6 ml ou plus, ou 12,5 ml ou plus est réduite d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 %, en comparaison à des témoins non traités,
(d) la proportion de sujets atteints de HIC chez lesquels le volume d'hématome a crû de 15 % ou plus, de 20 % ou plus, de 25 % ou plus, de 30 % ou plus, de 33 % ou plus, est réduite d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 %, en comparaison à des témoins non traités,
(e) la proportion de sujets atteints de HIC ayant un score de « spot sign » de 4 après traitement est réduite d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 %, en comparaison à des témoins non traités,
(f) la proportion de sujets atteints de HIC ayant un score de « spot sign » de 3 après traitement est réduite d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 %, en comparaison à des témoins non traités,
(g) la proportion de sujets atteints de HIC ayant un score de « spot sign » de 2 après traitement est réduite d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 %, en comparaison à des témoins non traités, ou
(h) la proportion de sujets atteints de HIC ayant un score de « spot sign » de 1 après traitement est réduite d'au moins environ 5 %, 10 %, 15 %, 20 %, 25 %, 30 %, 35 %, 40 %, 45 %, 50 %, 55 %, 60 %, 65 %, 70 %, 75 %, 80 %, 85 %, 90 %, ou 95 %, en comparaison à des témoins non traités.

8. Variant de facteur Xa (FXa) à utiliser selon la revendication 7(e) à (h), dans lequel le score de « spot sign » est déterminé pendant une période de temps après une première administration d'un variant FXa choisi dans le groupe constitué par 30 min, 45 min, 60 min, 75 min, 90 min, 2 h, 2,5 h, 3 h, 3,5 h, 4 h, 5 h, 6 h, 7 h, 8 h, 9 h, 10 h, 11 h, 12 h, 18 h, 24 h, 30 h, 48 h, 3 jours, 4 jours, 5 jours, 6 jours, 7 jours, 8 jours, 9 jours, 10 jours, 11 jours, 12 jours, 13 jours, 14 jours, 15 jours, 18 jours, 3 semaines, et 4 semaines.

9. Variant de facteur Xa (FXa) à utiliser selon l'une quelconque des revendications précédentes, dans lequel l'hémorragie intracérébrale se produit dans une région de l'encéphale choisie dans le groupe constitué par le cerveau, le lobe frontal, le lobe pariétal, le lobe occipital, le lobe temporal, le cervelet, le tronc cérébral, le mésencéphale, la protubérance annulaire, la substance médullaire, l'hypophyse, l'hypothalamus, le thalamus, l'hippocampe, l'amygdale, le claustrum, et les noyaux gris centraux.
